# EUROPEAN PATENT SPECIFICATION

(11) **EP 3 921 440 B1**
(45) Date of publication and mention of the grant of the patent: **27.03.2024**
(21) Application number: 20702483.7
(22) Date of filing: 05.02.2020
(51) Int. Cl.: C12Q 1/6811, C12Q 1/6853, C12Q 1/6841

(54) **METHOD AND PRODUCTS FOR PRODUCING FUNCTIONALISED SINGLE STRANDED OLIGONUCLEOTIDES**
VERFAHREN UND PRODUKTE ZUR HERSTELLUNG FUNKTIONALISIERTER EINZELSTRÄNGIGER OLIGONUKLEOTIDE
PROCÉDÉ ET PRODUITS DE PRODUCTION D'OLIGONUCLÉOTIDES MONOCATÉNAIRES FONCTIONNALISÉS

(30) Priority: 05.02.2019 GB 201901583
(43) Date of publication of application: 15.12.2021
(73) Proprietor: Moligo Technologies AB, 171 48 Solna (SE)
(72) Inventor: DUCANI, Cosimo, 171 77 Stockholm (SE); HOGBERG, Bjorn, 171 77 Stockholm (SE)
(74) Representative: Dehns
(86) International application number: PCT/EP2020/052868
(87) International publication number: WO 2020/161187

(56) References cited:
- WO-A1-2015/079042
- WO-A1-2015/179557
- WO-A2-99/09216
- WO-A2-2006/108423
- SMOLINA ET AL: "High-density fluorescently labeled rolling-circle amplicons for DNA diagnostics", ANALYTICAL BIOCHEMISTRY, ELSEVIER, AMSTERDAM, NL, vol. 347, no. 1, 1 December 2005 (2005-12-01), pages 152-155, XP005502686, ISSN: 0003-2697, DOI: 10.1016/J.AB.2005.06.002

## Description

The present invention relates to a method for producing functionalised single stranded oligonucleotides. In particular, the invention provides a method that utilises functionalised nucleotides in an enzyme-mediated rolling circle amplification (RCA) reaction to generate a plurality (e.g. a library) of functionalised oligonucleotides. A kit for use in the method is also provided.

Single stranded nucleotides are useful in a wide range of applications due to their ability to hybridise, via Watson-Crick base pairing, to complementary sequences, e.g. intermolecularly. In addition, single stranded oligonucleotides can hybridise to themselves, (i.e. intramolecular complementarity) so as to form complex topological geometries and molecular assemblies, including secondary structures known as aptamers. These aptamers can bind with high affinity to biological targets via non-covalent interactions to effect a range of different functions. The utility of oligonucleotides could be enhanced by the addition of functionalities to the nucleotides, particularly on the nucleobases (i.e. the addition of reactive chemical groups), which do not interfere with the Watson-Crick base pairing. However, the synthesis of such functionalised single stranded oligonucleotides can be problematic, and this has inhibited the growth of the aforementioned applications.

At present, single stranded oligonucleotides are typically produced using solid-phase synthesis, wherein nucleotides are added in a stepwise manner to a growing chain bound to a solid support. However, this method can be severely limited in terms of the length and accuracy of the oligonucleotides produced. The error rate in the production of oligonucleotides by solid-phase synthesis is significantly higher than the error rate of polymerase enzymes observed in nature, and increases dramatically with the length of the oligonucleotide, such that purities of only 70% are common in commercial oligonucleotides of around 50 residues in length. Moreover, the availability of single stranded oligonucleotides containing a high density of functionalised nucleotides has been restricted due to limitations in terms of length, quality and costs of solid-phase synthesis. In particular, the number of functionalised nucleotides that can be incorporated into single stranded oligonucleotides produced by solid-phase synthesis is limited, e.g. due to cross-reactions caused by the functional groups.

Many of the uses of synthetic oligonucleotides require high purity levels, meaning additional steps of purification via methods such as high performance liquid chromatography (HPLC) or polyacrylamide gel electrophoresis (PAGE) are often required to isolate the desired products of solid-phase synthesis reactions. This makes the current methods both labour intensive and expensive. Moreover, even after these additional purification steps, practical issues attributable to remaining impurities can still be observed.

In view of these issues with solid-phase methods, there is a desire for alternative methods of producing functionalised oligonucleotides, particularly cost-efficient methods capable of producing longer oligonucleotides with greater accuracy.

Modified (e.g. functionalised) nucleotides have previously been used in enzymatic reactions to produce oligonucleotides in the context of producing functionalised DNA via polymerase chain reactions (PCR) or primer extension (PE) reactions. These reactions necessitate the use of specific primers, which must then later be removed, and require not only that the modified nucleotides are incorporated by the polymerase, but also, in the case of PCR, that the functionalised products are recognised as templates. This can be problematic in the case of some modifications, and thus limits the utility of these methods. Moreover, the primers are synthesized by solid-phase methods and are not sequence verified, leading to the amplification of errors in the newly synthesized DNA. In addition, PCR-based methods result overwhelmingly in double stranded DNA products, and thus additional steps of elution and purification are necessary to obtain functionalised single stranded oligonucleotides.

WO 99/09216 describes methods for the production of detectably labelled oligonucleotides and multimers that are useful as diagnostic probes in medical, biological and chemical applications.

WO 2015/179557 describes methods for detecting the circular telomeric repeats that are indicative of an alternative lengthening of telomeres (ALT) mechanism exhibited by some cancer cells. The short circular telomeric repeats act as a template for a Rolling Circle Amplification reaction.

WO 2006/108423 describes methods for producing oligonucleotides using rolling circle replication, wherein synthesised multimeric oligonucleotides are reduced to mononucleotides using a nicking cassette.

The present inventors have previously described a method for enzymatic production of 'monoclonal stoichiometric' (MOSIC) single stranded DNA oligonucleotides from sequence-verified templates (Ducani et al., 2013, Nature Methods, 647-652). The inventors have surprisingly determined that the MOSIC method can be successfully adapted to produce single stranded functionalised oligonucleotides.

In a representative example, the method involves the design and preparation of a linear sequence comprising one or more oligonucleotide sequences, each bordered by hairpin sequences containing a restriction enzyme site. The linear sequence is then circularised into a double stranded rolling circle amplification (RCA) template, which is nicked and amplified by RCA in the presence of one or more functionalised nucleotides to produce a partially single stranded linear concatemer comprising functionalised nucleotides. The RCA product is then treated with a restriction enzyme which recognises the restriction site in the aforementioned hairpin regions, and cleaves the concatemer to release a plurality of functionalised single stranded oligonucleotides, which are then isolated or purified, i.e. to produce a library of functionalised oligonucleotides.

As shown in the Examples, the inventors have advantageously determined that, contrary to expectations, functionalised nucleotides were efficiently incorporated by DNA polymerases with strand displacement activity, and did not prevent the formation of hairpin structures or interfere with their stability or effective cleavage. Moreover, the inventors found that functionalised nucleotides may be utilised in the MOSIC method whilst still maintaining the beneficial characteristics associated with the method. For instance, in contrast to the aforementioned PCR-based methods, the present method does not require primers and can produce functionalised single stranded oligonucleotides directly. This avoids the need for any additional steps to allow for primer annealing or to convert double stranded products into single stranded oligonucleotides. Moreover, the present method does not require the functionalised nucleotides to be recognised as a template for further amplification, which means that greater numbers of functionalised residues can be incorporated.

Accordingly, the invention can be seen to provide the use of a circular DNA molecule comprising an oligonucleotide sequence bordered by cleavage domains in the production of an isolated or purified single stranded functionalised oligonucleotide, wherein the cleavage domains contain a sequence that is recognised by a cleavage enzyme, and wherein a cleavage domain comprises a sequence capable of forming a hairpin structure, wherein the double-stranded portion of the hairpin structure comprises the sequence that is recognised by a cleavage enzyme.

It will be evident that when the functionalised nucleotides are used in combination with the equivalent conventional nucleotide, the functionalised nucleotides may be incorporated randomly in the concatemer produced by the RCA reaction and thus cleavage of the concatemer results in a plurality (e.g. library) of single stranded functionalised oligonucleotides, i.e. where the functionalised nucleotides are incorporated in different positions in the oligonucleotides. It will be further evident that the diversity of the functionalised oligonucleotides produced may be increased by using a circular DNA molecule containing a plurality of oligonucleotide sequences, each bordered by cleavage domains. The oligonucleotide sequences may differ in their sequence and/or length. Additionally or alternatively, the diversity of the functionalised oligonucleotides may be increased by using a combination of functionalised nucleotides in the RCA reaction. Still further diversity may be introduced into the library of functionalised oligonucleotides by modifying the oligonucleotides after their synthesis, e.g. by conjugating molecules or components to the functionalised nucleotides in the oligonucleotides. Thus, it will be evident that the invention advantageously provides a new method for the enzymatic production of highly functionalised single stranded DNA oligonucleotides that may open new applications in DNA nanotechnology, nucleic acid imaging and bio-medicine. Thus, in one particular aspect, the present invention provides a method for producing single stranded functionalised oligonucleotides, said method comprising:
(a) providing a circular DNA molecule comprising an oligonucleotide sequence bordered by cleavage domains that contain a sequence that is recognised by a cleavage enzyme, wherein a cleavage domain comprises a sequence capable of forming a hairpin structure, wherein the double-stranded portion of the hairpin structure comprises the sequence that is recognised by a cleavage enzyme;
(b) performing a rolling circle amplification (RCA) reaction with the circular DNA molecule of (a) as a template and one or more functionalised nucleotides (dNTPs);
(c) enzymatically cleaving the product of the RCA reaction at the cleavage domains to release the single stranded functionalised oligonucleotides; and
(d) isolating or purifying the single stranded functionalised oligonucleotides.

As discussed in more detail below, the step of providing a circular DNA molecule may be achieved by any suitable means and may depend on the structure of the circular DNA molecule. In this respect, the circular DNA molecule may be a single stranded DNA molecule or a double stranded DNA molecule.

In embodiments where the circular DNA molecule is a double stranded molecule, it will be evident that the circular DNA molecule must be processed to provide an RCA template. Thus, in some embodiments, the method comprises an additional step of cleaving a single strand of the circular DNA molecule to provide an RCA template, before the RCA reaction is performed.

The present invention advantageously may be used to produce functionalised oligonucleotides comprising any oligonucleotide sequence. Thus, any suitable sequence may be used as the oligonucleotide sequence in the circular DNA molecule. By a suitable sequence, it is meant that the oligonucleotide sequence domain should not interfere with (i.e. inhibit or distort) the production or cleavage of the RCA product. For instance, in some embodiments the oligonucleotide sequence may be designed to avoid the generation of secondary structures that may inhibit the progression of, or result in the displacement of, the polymerase performing the RCA reaction. Nevertheless, in some embodiments, the oligonucleotide sequence may be, or may encode, an aptamer.

Moreover, the oligonucleotide sequence may be designed such that it does not hybridise specifically to the cleavage domains in the RCA product. Alternatively viewed, the cleavage domains that border the oligonucleotide sequence may be designed such that they do not hybridise specifically to the oligonucleotide sequence(s) in the RCA product.

In embodiments where the circular DNA molecule comprises a plurality of oligonucleotide sequences, each sequence may be designed such that it does not hybridise specifically to the other oligonucleotide sequences in the RCA product. However, in some embodiments, it may be desirable to produce functionalised oligonucleotides containing regions of complementarity, e.g. to enable said oligonucleotides to interact, particularly following their release from the RCA product. Thus, in some embodiments, the oligonucleotide sequences may be designed to facilitate the interaction (e.g. hybridisation) of functionalised oligonucleotides produced by the method insofar as such interactions do not interfere with the production or cleavage of the RCA product, i.e. the production of the functionalised oligonucleotides.

Thus, in some embodiments, the nucleic acid sequence of the oligonucleotide sequence of the circular DNA molecule has less than 80% sequence identity to the nucleic acid sequences in the cleavage domain and/or other oligonucleotide sequences in the circular DNA molecule. Preferably, the oligonucleotide sequence of the circular DNA molecule has less than 70%, 60%, 50% or less than 40% sequence identity to the nucleic acid sequences in the cleavage domain and/or other oligonucleotide sequences in the circular DNA molecule. Sequence identity may be determined by any appropriate method known in the art, e.g. the using BLAST alignment algorithm.

Thus, term "oligonucleotide sequence" is not particularly limiting and refers to the template sequence used to produce the functionalised single stranded oligonucleotide or oligonucleotides of the invention, or a complement thereof. In this respect, where the circular DNA molecule is single stranded, the sequence of the circular DNA molecule is the reverse complement of the repeated (tandem) sequence in the RCA product obtained in step (b). Where the circular DNA molecule is double stranded, it contains both the repeated sequence in the RCA product obtained in step (b) and its reverse complement. Accordingly, the step of processing the circular DNA to provide an RCA template may comprise a step of cleaving the strand of the circular DNA molecule containing the sequence that will be repeated in the RCA product.

The present invention may be used to produce functionalised single stranded oligonucleotides of any desired length. As shown in the Examples, the methods may be used to produce functionalised single stranded oligonucleotides that far exceed the length of oligonucleotides that can be accurately produced using solid-phase synthesis methods. Accordingly, an oligonucleotide sequence (and therefore functionalised oligonucleotide produced by the method) may be between about 6 up to about 10000 nucleotides in length. Thus, in some embodiments, the method may be viewed as the production of functionalised polynucleotides. In this respect, the boundary between the size of an "oligonucleotide" and "polynucleotide" is not well-defined in the art. For instance, a sequence of more than 400 nucleotides may be termed a polynucleotide. Accordingly, the terms oligonucleotide and polynucleotide are used interchangeably herein to refer to nucleotide sequences within the size range specified above. However, where the term "polynucleotide" is used, it will typically refer to sequences containing more than 400 nucleotides. Thus, in some embodiments, the method and use may be viewed as producing a plurality of single stranded functionalised DNA molecules.

In some embodiments, the oligonucleotide sequence may be from about 6 to about 750 nucleotides in length, including from about 6 to about 500 nucleotides in length, e.g., from about 6 to about 450 nucleotides in length, such as from about 8 to about 400 nucleotides in length, from about 8 to about 300 nucleotides in length, from about 8 to about 250 nucleotides in length, from about 10 to about 200 nucleotides in length, from about 10 to about 150 nucleotides in length, from about 12 to about 100 nucleotides in length, from about 12 to about 75 nucleotides in length, from about 14 to about 70 nucleotides in length, from about 14 to about 60 nucleotides in length, and so on. In some embodiments, the oligonucleotide sequence contains about 10-400, 11-390, 12-380, 13-370, 14-360 or 15-350 nucleotides.

As noted above, the invention is particularly effective in the production of longer oligonucleotides, e.g. comprising about 30 or more nucleotides, such as about 35, 40, 45, 50, 60, 70, 80, 90, 100 or more nucleotides. For instance, the oligonucleotides produced by the invention may contain about 30-1000, 40-900, 50-800, 60-700, 70-600, 80-500, 90-450 or 100-400 nucleotides. In some embodiments, the oligonucleotides (e.g. polynucleotides) produced by the invention may contain about 400-10000, 500-9000, 600-8000, 700-7000, 800-6000, 900-5000 or 1000-4000 nucleotides, e.g. comprising about 500, 1000, 1500, 2000, 2500, 3000, 3500 or more nucleotides.

In some embodiments, the circular DNA molecule comprises a plurality of oligonucleotide sequences, wherein each oligonucleotide sequence is bordered by cleavage domains. The oligonucleotide sequences may be the same or different from each other or a combination thereof. Thus, in some embodiments, the circular DNA molecule may comprise more than one copy of the same oligonucleotide sequence, e.g. 2, 3, 4, 5 etc. copies, as defined below. In some embodiments, the circular DNA molecule may comprise one copy each of a plurality of different oligonucleotide sequences, e.g. 2, 3, 4, 5 etc. different oligonucleotide sequences, as defined below. In some embodiments, the circular DNA molecule may comprise one or more copies of a plurality of different oligonucleotide sequences. As discussed further below, the present invention may be used to generate a plurality of functionalised oligonucleotides in controlled stoichiometry based on the number of copies of oligonucleotide sequences in the circular DNA molecule.

It will be evident that the plurality of oligonucleotide sequences in the circular DNA molecule may be present in any order. For instance, multiple copies of the same oligonucleotide sequence may be directly adjacent to each other in the circular DNA molecule (separated only by the cleavage domains that border the oligonucleotide sequences). Alternatively, multiple copies of the same oligonucleotide sequence may be interspersed with different oligonucleotide sequences. The circular DNA molecule may be designed (e.g. the order of the plurality of oligonucleotide sequences) to avoid or minimise interactions between the repeated sequences in the RCA product that may interfere with the production or cleavage of the RCA product as defined above.

As used herein, the term "plurality" means two or more, e.g. at least 2, 3, 4, 5, 6, 7, 8, 9, 10, 20 or 30 or more, such as 50, 100, 150, 200, 250 or more depending on the context of the invention. For instance, the circular DNA molecule may contain at least 2, 3, 4, 5, 6, 7, 8, 9, 10, 20 or 30 or more oligonucleotide sequences, such as 2-100, 3-90, 4-80, 5-70, 6-60, 7-50, 8-40, 9-30 or 10-20 oligonucleotide sequences. In some embodiments, the method of the invention may produce at least 2, 3, 4, 5, 6, 7, 8, 9, 10, 20 or 30 or more functionalised single stranded oligonucleotides, such as 50, 100, 150, 200, 250 or more, i.e. oligonucleotides with different sequences and/or structures. In this respect, different functionalised oligonucleotides may be produced from the same template oligonucleotide sequence because functionalised nucleotides may be incorporated randomly in the RCA product, i.e. when the functionalised nucleotides are present in a relative amount of less than 100% as described above. Moreover, if the circular DNA molecule contains a plurality of different oligonucleotide sequences, each template will result in a plurality of different functionalised single stranded oligonucleotides. Furthermore, as an RCA reaction may utilise a plurality of circular DNA molecules, the reaction will result in a plurality of each functionalised single stranded oligonucleotide, e.g. 10³, 10⁴, 10⁵, 10⁶, 10⁷, 10⁸, 10⁹, 10¹⁰ or more copies of each functionalised single stranded oligonucleotide.

The term "different" refers to oligonucleotide sequences or functionalised single stranded oligonucleotides comprising one or more different nucleotides. Thus, different oligonucleotide sequences may differ by one or more, e.g. 2, 3, 4, 5, 6, 7, 8, 9, 10 or more, nucleotides, e.g. 20, 30, 40, 50, 60, 70, 80, 90 or more nucleotides. The differences may be in the length and/or sequence of the oligonucleotide sequence. Alternatively viewed, the different oligonucleotide sequences have less than 100% sequence identity each other, such as less than 99%, 98%, 97%, 96%, 95%, 90%, 85%, 80%, 70%, 60%, 50% to each other. Different functionalised single stranded oligonucleotides may comprise the same nucleotide sequences but differ in the position of functionalised nucleotides within the oligonucleotide or the type of functionalised nucleotide at a particular position (e.g. where a combination of functionalised nucleotides is used in the RCA reaction). Alternatively or additionally, different functionalised single stranded oligonucleotides may differ in the oligonucleotide sequence as defined above.

The present invention may therefore be used to produce a number of different single stranded functionalised oligonucleotides simultaneously. In particular, by altering the sequence of the circular DNA molecule, specifically, by controlling the number of copies of each different oligonucleotide sequence that are present, it is possible to control the stoichiometry of the single stranded functionalised oligonucleotides that are ultimately produced by the present method. However, as the functionalised single stranded oligonucleotides derived from each oligonucleotide sequence may differ (due to the random incorporation of functionalised nucleotides in the RCA product), the stoichiometry of the functionalised single stranded oligonucleotides may be controlled with respect to the number of oligonucleotides based on a particular oligonucleotide sequence. The circular DNA molecule may therefore comprise a plurality of copies of a plurality of different oligonucleotide sequences.

The term "cleavage domain" as used herein typically refers to a domain within the circular DNA molecule that results in a domain within the RCA product that can be enzymatically cleaved specifically to release the functionalised single stranded oligonucleotides.

"Cleavage" includes any means of breaking a covalent bond. Thus, in the context of the invention, cleavage involves cleavage of a covalent bond in a nucleotide chain (i.e. strand cleavage or strand scission), for example by cleavage of a phosphodiester bond.

Thus, a cleavage domain comprises a sequence that is recognised by one or more enzymes capable of cleaving a nucleic acid molecule, i.e. capable of breaking the phosphodiester linkage between two or more nucleotides.

Thus, step (c) comprises contacting the product of the RCA reaction with a cleavage enzyme.

For instance, a cleavage domain may comprise a restriction endonuclease (restriction enzyme) recognition sequence. Restriction enzymes cut double-stranded or single stranded DNA at specific recognition nucleotide sequences known as restriction sites and suitable enzymes are well-known in the art. For example, it may be particularly advantageous to use rare-cutting restriction enzymes, i.e. enzymes with a long recognition site (at least 8 base pairs in length), to facilitate the design of the oligonucleotide sequence(s) in the circular DNA molecule, e.g. to avoid the inclusion of a cleavage recognition site within the oligonucleotide sequence(s).

In some embodiments, a cleavage domain may comprise a sequence that is recognised by a type II restriction endonuclease, more preferably a type Ils restriction endonuclease. While any suitable cleavage domain and cleavage enzyme may be used in the invention, in some embodiments the cleavage enzyme that recognises a cleavage domain bordering the oligonucleotide sequences may be BseGl, BtsCl or an isoschizomer thereof, e.g. BstF5I or FokI. Other representative enzymes that may be used include BsrDl, Btsl, BtslMutl, Mlyl or isoschizomers thereof.

Thus, the step of cleaving the product of the RCA reaction comprises contacting the RCA product with a cleavage enzyme under suitable conditions to selectively cleave the RCA product in the cleavage domains.

A cleavage domain comprises or consists of a sequence capable of forming a hairpin structure. A hairpin structure may also be known as a hairpin-loop or a stem-loop and these terms are used interchangeably herein. A hairpin is an intramolecular base-pairing pattern that can occur in a single-stranded DNA or RNA molecule. A hairpin occurs when two regions of the same strand, usually complementary in nucleotide sequence when read in opposite directions, base-pair (hybridise) to form a double stranded stem (a duplex) and an unpaired, i.e. single-stranded, loop. The resulting structure can be described as lollipop-shaped.

Thus, as the cleavage domain comprises or consists of a sequence capable of forming a hairpin structure, the cleavage domain comprises sequences that are self-complementary. As the RCA product extends, the hybridisation of these self-complementary regions results in a hairpin structures, wherein the double-stranded portion of the hairpin structure comprises a sequence that is recognised by a cleavage enzyme. Thus, cleavage of the double-stranded portion of the hairpin structures in the RCA product results in the release of the functionalised single stranded oligonucleotides and hairpin structures (i.e. oligonucleotides that form the hairpin structures).

The term "hybridisation" or "hybridises" as used herein refers to the formation of a duplex between nucleotide sequences which are sufficiently complementary to form duplexes via Watson-Crick base pairing. Two nucleotide sequences are "complementary" to one another when those molecules share base pair organization homology. "Complementary" nucleotide sequences will combine with specificity to form a stable duplex under appropriate hybridisation conditions. For instance, two sequences are complementary when a section of a first sequence can bind to a section of a second sequence in an antiparallel sense wherein the 3'-end of each sequence binds to the 5'-end of the other sequence and each A, T(U), G and C of one sequence is then aligned with a T(U), A, C and G, respectively, of the other sequence. RNA sequences can also include complementary G=U or U=G base pairs. Thus, two sequences need not have perfect homology to be "complementary" under the invention. Usually two sequences are sufficiently complementary when at least about 90% (preferably at least about 95%) of the nucleotides share base pair organization over a defined length of the molecule.

Upon cleavage of the RCA product, the functionalised oligonucleotides are released. The functionalised oligonucleotides that are released may consist only of the oligonucleotide sequence (i.e. with no additional nucleotides), or they may comprise one or more additional nucleotides from the cleavage domains that border the oligonucleotide sequences at one or both ends. Thus, in some embodiments, the functionalised oligonucleotides may comprise 1, 2, 3, 4, 5, 6, 7, 8, 9 or 10 or more nucleotides from the cleavage domains at one or both ends. Preferably, the sequence of the circular DNA molecule is designed such that when the RCA product is cleaved, the functionalised oligonucleotides that are released do not contain any additional nucleotides from the cleavage domains. Alternatively viewed, the cleavage domains that border the oligonucleotide sequence(s) may be arranged in the circular DNA molecule such that their cleavage in the RCA product results in the release of functionalised oligonucleotides that do not contain any additional nucleotides from the cleavage domains.

As cleavage enzymes may cleave a nucleic acid molecule at a position outside of the cleavage enzyme recognition sequence, there may be one or more nucleotides between a cleavage domain and an oligonucleotide sequence. Alternatively viewed, the cleavage domain may contain nucleotide sequences in addition to the cleavage enzyme recognition sequence to ensure that cleavage results in the release of the complete functionalised single stranded oligonucleotides, preferably without any additional nucleotides (e.g. nucleotides that form part of the cleavage domains).

Thus, the term "bordered" refers to cleavage domains that are directly or indirectly adjacent to the oligonucleotide sequence. Alternatively viewed, the cleavage domains are positioned at either end of the oligonucleotide sequence, i.e. the cleavage domains are upstream and downstream (at the 5' and 3' ends) of the oligonucleotide sequence. In some embodiments, the cleavage site of the cleavage domains (e.g. the site at which a cleavage enzyme cleaves a cleavage domain) is directly adjacent to the end of the oligonucleotide sequence it borders. In some embodiments, the oligonucleotide sequence and cleavage domain sequence may overlap, i.e. the end of the oligonucleotide sequence may form part of the cleavage domain, e.g. when the cleavage site is an internal site within the cleavage domain, i.e. the cleavage domains may form the ends or part of the ends of the oligonucleotide sequence. Thus, in some embodiments, there may be one or more, e.g. 1, 2, 3, 4, 5, 6, 7, 8, 9 or 10 or more, nucleotides between the cleavage domain and the oligonucleotide sequence (i.e. between the ends of the sequences). In some embodiments, the cleavage domain and the oligonucleotide sequence may overlap one or more, e.g. 1, 2, 3, 4, 5, 6, 7, 8, 9 or 10 or more, nucleotides.

The size of the cleavage domains in the circular DNA molecule is not particularly limited and will depend on the type of cleavage domain, as described above. The cleavage domain may be designed or selected such that the length of the cleavage domain is different from the length of the oligonucleotide sequences, to allow for the functionalised single stranded oligonucleotide sequences to be easily purified once the RCA product has been cleaved at the cleavage domain. Thus, in some embodiments, the cleavage domain(s) may be selected to be shorter than the oligonucleotide sequence in the circular DNA molecule. If the circular DNA molecule contains a plurality of oligonucleotide sequences of different lengths, the cleavage domain(s) may be selected to be shorter than the shortest oligonucleotide sequence in the circular DNA molecule. In other embodiments, the cleavage domain(s) may be selected to be longer than the oligonucleotide sequence in the circular DNA molecule. If the circular DNA molecule contains a plurality of oligonucleotide sequences of different lengths, the cleavage domain(s) may be selected to be longer than the longest oligonucleotide sequence in the circular DNA molecule. In some embodiments, the length of the cleavage domain(s) and the oligonucleotide sequences differ by at least 2 nucleotides, such as at least 3, 4, 5, 6, 7, 8, 9 or 10 nucleotides.

If the desired oligonucleotide sequences and the cleavage domains are of approximately the same length, the cleavage domains may be extended so that they can be separated from the final single stranded functionalised oligonucleotides by size. For example, additional regions of self-complementarity may be added to the ends of the cleavage domains so as to extend the length of the stem region of the hairpin structure. Alternatively or additionally, nucleotides may be added to the loop region of the hairpin structure.

In some embodiments, cleavage domains may be about 4-50 nucleotides in length, such as about 5-45, 6-40, 7-35 or 8-30 nucleotides in length. In some embodiments they may range from about 10 to 25 nucleotides in length, including from about 12 to 22 or from about 14 to 20. However, it will be evident that any suitable length of cleavage domain may be used in the invention as long as it meets the functional requirements described above.

The cleavage domains that border the oligonucleotide sequences may be the same or different from each other. Advantageously, the cleavage domains that border the oligonucleotide sequence(s) are the same such that a single cleavage step is sufficient to release all of the functionalised single stranded oligonucleotides. For instance, in some embodiments, the step of cleaving the RCA product comprises contacting the RCA product with a single cleavage enzyme under conditions suitable to cleave the cleavage domains in the RCA product.

Suitable conditions to cleave the cleavage domains in the RCA product will be dependent on the means used to achieve cleavage. For instance, where cleavage is achieved using a restriction endonuclease, conditions will differ depending on the enzyme selected and suitable conditions are well-known in the art, e.g. the cleavage step may follow the manufacturer's instructions. An example of a range suitable conditions that may be used in the cleavage step is set out below.

A cleavage enzyme, e.g. a restriction endonuclease, may specifically bind to its cleavage recognition site and selectively (e.g. specifically) cleave the nucleic acid in a variety of buffers, such as phosphate buffered saline (PBS), 4-(2-hydroxyethyl)-1-piperazineethanesulfonic acid (HEPES), HEPES buffered saline (HBS), and Tris buffered saline (TBS), both with and without EDTA. Cleavage may occur in a pH range of about 3.0-10.0, e.g. 4.0-9.0, 5.0-8.0, over a wide range of temperatures, e.g. 0-70 °C. The skilled person would readily be able to determine other suitable conditions.

The method of the invention comprises a step of performing rolling circle amplification using the circular DNA molecule as a template. Rolling-circle amplification (RCA) is well-known in the art, being described in Dean et al., 2001 (Rapid Amplification of Plasmid and Phage DNA Using Phi29 DNA Polymerase and Multiply-Primed Rolling Circle Amplification, Genome Research, 11, pp. 1095-1099). In brief, RCA relates to the synthesis of nucleic acid molecules using a circular single stranded nucleic acid molecule, e.g. a circle or circular oligonucleotide, as rolling circle template (an RCA template) and a strand-displacing polymerase to extend a primer which is hybridised to the template. The addition of a polymerase and nucleotides starts the synthesis reaction, i.e. polymerization. As the rolling circle template is endless, the resultant product is a long single stranded nucleic acid molecule composed of tandem repeats that are complementary to the rolling circle template.

A typical RCA reaction mixture includes a circular DNA molecule and one or more primers that are employed in the primer extension reaction, e.g. RCA may be templated by a single primer to generate a single concatemeric product or multiple primers, each annealing to a different region of the circular oligonucleotide to produce multiple concatemeric products per circle. The oligonucleotide primers with which the circular nucleic acid may be contacted will be of sufficient length to provide for hybridisation to the circular DNA molecule under annealing conditions. In some embodiments, the primer may be provided by cleaving (e.g. nicking) a single strand of a double stranded circular DNA molecule provided in step (a).

In addition to the above components, the reaction mixture used in the invention typically includes a polymerase (defined further below, e.g. phi29 DNA polymerase), one or more functionalised nucleotides, one or more conventional nucleotides and other components required for a DNA polymerase reaction as described below. The desired polymerase activity may be provided by one or more distinct polymerase enzymes.

The RCA reaction mixture may further include an aqueous buffer medium that includes a source of monovalent ions, a source of divalent cations and a buffering agent. Any convenient source of monovalent ions, such as KCI, K-acetate, NH₄-acetate, K-glutamate, NH₄Cl, ammonium sulphate, and the like may be employed. The divalent cation may be magnesium, manganese, zinc and the like, where the cation will typically be magnesium. Any convenient source of magnesium cation may be employed, including MgCl₂, Mg-acetate, and the like. The amount of Mg²⁺ present in the buffer may range from 0.5 to 10 mM, but will preferably range from about 3 to 6 mM, and will ideally be at about 5 mM. Representative buffering agents or salts that may be present in the buffer include Tris, Tricine, HEPES, MOPS and the like, where the amount of buffering agent will typically range from about 5 to 150 mM, usually from about 10 to 100 mM, and more usually from about 20 to 50 mM, where in certain preferred embodiments the buffering agent will be present in an amount sufficient to provide a pH ranging from about 6.0 to 9.5. Other agents which may be present in the buffer medium include chelating agents, such as EDTA, EGTA and the like.

In embodiments in which a single strand of the double stranded circular DNA molecule is cleaved (e.g. nicked) to provide the RCA template (and the primer for the RCA reaction), it may be useful to include a single stranded binding protein in the RCA reaction mixture. For example, E. coli single stranded DNA binding protein has been used to increase the yield and specificity of primer extension reactions and PCR reactions. (U.S. Pat. Nos. 5,449,603 and 5,534,407). The gene 32 protein (single stranded DNA binding protein) of phage T4 apparently improves the ability to amplify larger DNA fragments (Schwartz, et al., Nucl. Acids Res. 18: 1079 (1990)), it enhances DNA polymerase fidelity (Huang, DNA Cell. Biol. 15: 589-594 (1996)) and, most importantly, it prevents DNA polymerase from switching templates, e.g. to the already produced single stranded DNA, and synthesizing double stranded DNA (Ducani et al. Nucl. Acids Res. 42: 10596 (2014)). When employed, such a protein will be used to achieve a concentration in the reaction mixture that ranges from about 0.01 ng/µL to about 1 µg/µL; such as from about 0.1 ng/µL to about 100 ng/µL; including from about 1 ng/µL to about 10 ng/µL.

The RCA reaction ultimately produces a polynucleotide product comprising adjacent (tandem) repeats of the complementary sequence of the circular DNA molecule. This product may be known as a concatemer, an RCA product or "RCP". The RCA product therefore comprises a linear sequence made up of oligonucleotide sequences (or more particularly the reverse complement of the oligonucleotide sequences of the circular DNA molecule template) comprising functionalised nucleotides, bordered by cleavage domains.

As noted above, where the circular DNA molecule is single stranded, the RCA reaction mixture may comprise one or more oligonucleotide primers, which initiate the RCA polymerisation reaction. The primers will be of sufficient length to provide for hybridisation to the circular DNA molecule under annealing conditions. The primers will generally be at least 10 nucleotides in length, usually at least 15 nucleotides in length and more usually at least 16 nucleotides in length and may be as long as 30 nucleotides in length or longer, where the length of the primers will generally range from 18 to 50 nucleotides in length, usually from about 20 to 35 nucleotides in length.

The primers may anneal to any region within the circular DNA molecule. In some embodiments, the circular DNA molecule may comprise a specific domain (an RCA primer binding site) to which the primer may hybridise. In a representative embodiment, the circular DNA molecule may comprise a sequence between the cleavage domains that border the oligonucleotide sequence, which is not the oligonucleotide sequence and which may function as the RCA primer binding site. The RCA primer binding site may be designed to be of a different length to the oligonucleotide sequence, akin to the cleavage domains discussed above, to ensure that it can be readily separated from the functionalised single stranded oligonucleotides upon cleavage of the RCA product. It will be evident that in a circular DNA molecule comprising a plurality of oligonucleotide sequences bordered by cleavage domains, the RCA primer binding site may be between any two cleavage domains.

The term "annealing conditions" refers to the conditions under which two nucleic acid molecules comprising complementary nucleotide sequences will specifically hybridise to each other. Various parameters affect hybridisation including temperature, salt concentration, nucleic acid concentration, composition and length, and buffer composition. The skilled person readily can determine suitable annealing conditions for a particular primer/template combination for an RCA reaction as a matter of routine.

As noted above, in some embodiments, the circular DNA molecule is double stranded and the method comprises an additional step of cleaving a single strand of the circular DNA molecule to provide an RCA template, before the RCA reaction is performed. It will be evident that the step of cleaving a single strand of the circular DNA molecule may replace the step of providing a primer to initiate the RCA reaction, i.e. the cleaved strand functions as the RCA primer. Thus, alternatively viewed, the method comprises an additional step of cleaving a single strand of the circular DNA molecule to provide an RCA template and primer, before the RCA reaction is performed, i.e. the introduction of a single strand break in the circular DNA molecule creates a 3' end which can act as a primer for the RCA reaction. However, in some embodiments, it may be advantageous to provide one or more RCA primers in the reaction mixture in addition to cleaving one strand of the double stranded circular DNA molecule, e.g. to increase the number of RCA products obtained per circle.

In some embodiments, the step of cleaving a single strand of the circular DNA molecule to provide an RCA template comprises cleaving a single strand of the circular DNA molecule with a cleavage enzyme. This cleavage of a single strand of the circular DNA molecule results in a single strand break (a nick).

In some embodiments, it may be advantageous to cleave a single strand of the circular DNA molecule multiple times in close proximity, in order to facilitate the binding of the DNA polymerase to the 3' end generated by the cleavage. Accordingly, a single strand of the circular DNA molecule may be cleaved two or more times, i.e. two or more nicks may be generated, in close proximity to each other. Preferably, the nicks are generated within 20 nucleotides, more preferably within 10 nucleotides, more preferably within 5 nucleotides of each other, e.g. within 1, 2, 3, 4 or 5 nucleotides of each other.

In some embodiments, the cleavage enzyme used to cleave one strand of the double stranded circular DNA molecule is a nickase. Nickases are endonucleases which cleave only a single strand of a DNA duplex. Some nickases introduce single-stranded nicks only at particular sites on a DNA molecule, by binding to and recognizing a particular nucleotide recognition sequence. A number of naturally-occurring nickases have been discovered, of which at present the sequence recognition properties have been determined for at least four. Nickases are described in U.S. Patent No. 6,867,028, and any suitable nickase may be used in the methods of the invention. In some embodiments, the cleavage enzyme (nickase) may be Nb.BsrDl, Nt.BspQl or a combination thereof.

In some embodiments that utilise a nickase enzyme, the nickase enzyme may be removed from the assay or inactivated following cleavage of the circular DNA molecule to prevent unwanted cleavage of RCA products.

The cleavage enzyme that cleaves a single strand of the circular DNA molecule (e.g. nickase) may cleave at any site within the circular DNA molecule. In some embodiments, the circular DNA molecule may comprise a specific domain (a single strand cleavage site or domain, e.g. a nickase site) at which the cleavage enzyme may act. In a representative embodiment, the circular DNA molecule may comprise a sequence between the cleavage domains, which is not the oligonucleotide sequence and which may function as the single strand cleavage site or domain. The fact that the sequence recognised by the cleavage enzyme (the single strand cleavage site or domain) is not in the oligonucleotide sequence ensures that the oligonucleotide produced from the 5' end of the RCA product is not truncated. The single strand cleavage site or domain may be designed to be of a different length to the oligonucleotide sequence, akin to the cleavage domains and RCA primer binding site discussed above, to ensure that it can be readily separated from the functionalised single stranded oligonucleotides upon cleavage of the RCA product. Thus, in some embodiments, the RCA primer binding site also functions as the single strand cleavage site or domain or *vice versa.*

Any DNA polymerase with at least some strand displacement activity may be used in the RCA reaction of the invention. Strand displacement activity ensures that once the polymerase has extended around the circular DNA molecule, it can displace the primer sequence and the elongating product and continue to "roll" around the template. In embodiments where the nicked strand of the circular DNA molecule provides the primer for RCA extension, the strand displacement activity ensures that the polymerase can displace the nicked strand. Suitable DNA polymerase enzymes with at least some strand displacement activity include phi29 DNA polymerase, E.coli DNA polymerase I, Bsu DNA polymerase (large fragment), Bst DNA polymerase (large fragment) and Klenow fragment. As used herein, the term "DNA polymerase" includes not only naturally occurring enzymes but also all such modified derivatives, including also derivatives of naturally occurring DNA polymerase enzymes. For instance, in some embodiments, the DNA polymerase may have been modified to remove 5'-3' exonuclease activity.

Particularly preferred DNA polymerase enzymes for use in the invention include phi29 DNA polymerase, Bst DNA polymerase and derivatives, e.g. sequence-modified derivatives, or mutants thereof.

Sequence-modified derivatives or mutants of DNA polymerase enzymes include mutants that retain at least some of the functional activity, e.g. DNA polymerase activity and at least some strand displacement activity, of the wild-type sequence. Mutations may affect the activity profile of the enzymes, e.g. enhance or reduce the rate of polymerisation, under different reaction conditions, e.g. temperature, template concentration, primer concentration etc. Mutations or sequence-modifications may also affect the exonuclease activity and/or thermostability of the enzyme.

As noted above, the RCA reaction of the present method is typically conducted using a mixture of functionalised and conventional nucleotides.

The term "conventional nucleotides" as used herein refers to deoxynucleotides comprising one of the four bases found in DNA; adenine, guanine, cytosine and thymine. The term "conventional nucleotides" thus encompasses, for example, dATP, dGTP, dCTP and dTTP. Whilst uracil is not typically found in DNA naturally, dUTP readily may be used instead of, or in addition to, dTTP. Thus, in the context of the present invention, dUTP may be viewed as a "conventional" nucleotide. Usually the reaction mixture will include at least three of the four different types of dNTPs corresponding to the four naturally occurring bases present, i.e. dATP, dTTP, dCTP and dGTP. However, as noted above, dUTP may be used instead of, or in addition to, dTTP. Moreover, in some embodiments, the reaction mixture may include four or five dNTPs, i.e. dATP, dCTP, dGTP, dTTP and/or dUTP. In the subject methods, each dNTP will typically be present in an amount ranging from about 10 to 5000 µM, usually from about 20 to 1000 µM. Each dNTP may be present in the different amounts or an equal amount of each dNTP may be used.

The terms "functionalised nucleotides" or "functionalised dNTPs" refer to nucleotides that comprise a modification relative to unmodified conventional nucleotides, wherein said modification provides said functionalised nucleotides and/or oligonucleotides comprising at least one functionalised nucleotide with additional or alternative properties or characteristics, i.e. relative to the corresponding conventional nucleotide. For instance, the modification may render the nucleotide detectable, e.g. by the incorporation of a label, or capable of interacting and/or reacting with another component, i.e. a component with which the corresponding conventional nucleotide does not interact or react. In some embodiments, the modification may render the oligonucleotide containing the nucleotide resistant to degradation, e.g. chemical and/or enzymatic degradation (e.g. nuclease degradation), or may alter the metabolism of the nucleotide.

The terms "functionalised single stranded oligonucleotide", "single stranded functionalised oligonucleotide" and "functionalised oligonucleotide" are used interchangeably herein and refer to a single stranded oligonucleotide containing at least one functionalised nucleotide. Thus, a functionalised oligonucleotide has additional or alternative properties or characteristics relative to the corresponding oligonucleotide containing only conventional nucleotides. For instance, the incorporation of one or more functionalised nucleotides may render the oligonucleotide detectable, e.g. by the incorporation of a label, or capable of interacting and/or reacting with another component, i.e. a component with which the corresponding oligonucleotide containing only conventional nucleotides does not interact or react. In some embodiments, the modification may render the oligonucleotide resistant to degradation, e.g. chemical and/or enzymatic degradation (e.g. nuclease degradation), or may alter the metabolism of the oligonucleotide. In some embodiments, the modification may improve the stability of the oligonucleotide, e.g. improve the stability of duplexes formed by the oligonucleotide, such as the thermostability of the duplexes (e.g. melting temperature). In some embodiments, the incorporation of one or more functionalised nucleotides may render the oligonucleotide capable of forming secondary or tertiary structures that are not formed by a corresponding oligonucleotide containing only conventional nucleotides.

Accordingly, it will be understood that in the context of a functionalised single stranded oligonucleotide, the term "single stranded" refers to an oligonucleotide which is single stranded under denaturing conditions, e.g. following the application of heat or suitable chemical denaturing agents, i.e. an oligonucleotide with only one continuous backbone (one strand). As noted above, this does not preclude a functionalised single stranded oligonucleotide from forming secondary or tertiary structures. For example, the functionalised single stranded oligonucleotide may comprise regions of self-complementarity, and thus may be capable of forming a hairpin or stem-loop structure, mediated by one region of the functionalised single stranded oligonucleotide hybridising to a complementary region elsewhere in the same oligonucleotide.

Functionalisation of a conventional nucleotide may be achieved by alteration of, or modification to, the structure of any part of the nucleotide. Thus, a functionalised nucleotide may contain a modification, e.g. a chemical modification, on the nucleobase, sugar or group involved in the internucleotide linkage. In some preferred embodiments, the functionalised nucleotide contains a modification, e.g. a chemical modification, on the nucleobase. Modifications at various positions are described in more detail below and it is contemplated that any of the specific positions described below may be modified with any of functional groups described below.

For instance, the substitution of the C5 position in pyrimidines (i.e. cytosine, thymine and uracil) with a group that is small, rigid and hydrophobic may improve base stacking interactions of, and/or stabilize duplexes formed by, oligonucleotides comprising functionalised nucleotides containing the substituted pyrimidines. A small, rigid and hydrophobic group may be an alkynyl group, e.g. methyl, ethynyl, propynyl, or a halogen group, e.g. fluoro, chloro or bromo. Thus, in some embodiments, the functionalised nucleotide comprises a pyrimidine with a substitution at the C5 position, e.g. an alkynyl group or halogen as defined above.

In some embodiments, a modification that may render the nucleotide detectable may involve the incorporation of a label into the nucleotide. Any label may find utility in the invention and may be a directly or indirectly signal giving molecule. For instance, directly signal giving labels may be fluorescent molecules, i.e. the functionalised nucleotides may be fluorescently labelled nucleotides. Indirectly signal giving labels may be, for example, biotin molecules, i.e. the labelled nucleotides may be biotin labelled nucleotides, which require additional steps to provide a signal, e.g. the addition of streptavidin conjugated to an enzyme which may act on a chemical substrate to provide a detectable signal, e.g. a visibly detectable colour change. In some embodiments, the label is incorporated in (conjugated to) the nucleobase.

Thus, in some embodiments, the functionalised nucleotide comprises a biotin group conjugated to a nucleobase. In some embodiments, the biotin group may be conjugated to the nucleobase indirectly, e.g. via a linker or linking domain and a suitable linker may be readily selected from those well-known in the art. For instance, the linker may be selected to facilitate the interaction between biotin and streptavidin, i.e. to minimise or prevent steric hindrance. In some embodiments, the biotin group is conjugated to a pyrimidine at the C5 position. In a representative embodiment, the biotin containing functionalised nucleotide may be Biotin-16-Aminoallyl-2'-dUTP, Biotin-16-Aminoallyl-2'-dTTP or Biotin-16-Aminoallyl-2'-dCTP.

In some embodiments, the nucleotide may be labelled with a sterol group, i.e. the nucleotide may comprise a sterol group. In some embodiments, the nucleotide may be labelled with or may comprise a cholesterol group.

A directly detectable label is one that can be directly detected without the use of additional reagents, while an indirectly detectable label is one that is detectable by employing one or more additional reagents, e.g. where the label is a member of a signal producing system made up of two or more components. In many embodiments, the label is a directly detectable label, where directly detectable labels of interest include, but are not limited to: fluorescent labels, coloured labels, radioisotopic labels, chemiluminescent labels, and the like. Any spectrophotometrically or optically-detectable label may be used. In other embodiments the label may provide a signal indirectly, i.e. it may require the addition of further components to generate signal. For instance, the label may be capable of binding a molecule that is conjugated to a signal giving molecule.

In some embodiments, the functionalised nucleotide is a fluorescently labelled nucleotide. Whilst fluorescent labels require excitation to provide a detectable signal, as the source of excitation is derived from the instrument/apparatus used to detect the signal, fluorescent labels may be viewed as directly signal giving labels.

Fluorescent molecules that may be used to label nucleotides are well known in the art. Fluorophores have been identified with excitation and emission spectra ranging from UV to near IR wavelengths. Thus, the fluorophore may have an excitation and/or emission wavelength in the UV, visible or IR spectral range.

The fluorophore may be a protein, peptide, small organic compound, synthetic oligomer or synthetic polymer. In some embodiments, the fluorophore is a small organic compound, e.g. an organic compound with a molecular weight of 5000 Da or less. Thus, in some embodiments, the fluorophore has a molecular weight of 4000 Da or less, such as 3500 Da, 3000 Da, 2500 Da, 2250 Da, 2000 Da, 1900 Da, 1800 Da, 1700 Da, 1600 Da, 1500 Da or less.

Thus, the fluorophore may be a xanthene derivative (e.g. fluorescein, rhodamine, Oregon green, eosin, Texas red), a cyanine derivative (e.g. cyanine, indocarbocyanine, oxacarbocyanine, thiacarbocyanine, merocyanine), a squaraine derivative (e.g. ring-substituted squaraine, Seta, SeTau), a naphthalene derivative (e.g. dansyl or prodan derivative), a coumarin derivative, an oxadiazole derivative (such as pyridyloxazole, nitrobenzoxadiazole and benzoxadiazole), an anthracene derivative (such as an anthraquinone, including DRAQ5, DRAQ7 and CyTRAK Orange), a pyrene derivative (e.g. cascade blue), an oxazine derivative (e.g. Nile red, Nile blue, cresyl violet, oxazine 170), an acridine derivative (such as proflavin, acridine orange, acridine yellow), an arylmethine derivative (e.g. auramine, crystal violet, malachite green) or a tetrapyrrole derivative (such as porphyrin, phthalocyanine, bilirubin).

Specific examples of fluorophores or fluorophore series that may find utility in the present invention include Alexa Fluors (such as Alexa Fluor 488, Alexa Fluor 647 etc.), Atto, Cyanine (Cy), indocyanine, sulfocyanine, DyLight, Abberior STAR, Chromeo, Oregon Green, Fluorescein, Texas red, Rhodamine, Silicon-rhodamine (SiR), Squaraine, FluoProbes, Tetrapyrrole, Bodipy, HiLyte, Quasar, CAL fluor, Coumarin, Seta, CF, Tracy, IRDye, CruzFluor, Tide Fluor, Oyster, iFluor, Chromis and Brilliant Violet and fluorescent derivatives or analogues thereof.

In some embodiments, the functionalised nucleotide contains a cyanine fluorescent label, such as Cy3. In some particular embodiments, the functionalised nucleotide is dATP labelled with Cy3, e.g. 7-Propargylamino-7-deaza-ATP-Cy3.

In some embodiments, the functionalised nucleotide contains an atto fluorescent label, such as atto-488. In some particular embodiments, the functionalised nucleotide is dATP labelled with atto-488, e.g. 7-Propargylamino-7-deaza-ATP-Atto-488.

In some embodiments, a modification that may render the nucleotide reactive involves the incorporation of a reactive group, e.g. a group capable of forming a covalent bond with another chemical group, e.g. a chemical group on a molecule or component to be conjugated to the functionalised oligonucleotide, such as a label as defined herein. Potential reactive groups include nucleophilic functional groups (alkynes, alkenyls, amines, alcohols, thiols, hydrazides, azides), electrophilic functional groups (aldehydes, esters, vinyl ketones, epoxides, isocyanates, maleimides), functional groups capable of cycloaddition reactions, forming disulfide bonds, or binding to metals. Specific examples include ethyne (acetylene), propyne, 1-butyne, 2-butyneazide, vinyl (ethenyl), propenyl, 1-butenyl, primary and secondary amines, hydroxamic acids, N-hydroxysuccinimidyl esters, N-hydroxysuccinimidyl carbonates, oxycarbonylimidazoles, nitrophenylesters, trifluoroethyl esters, glycidyl ethers, vinylsulfones, azides and maleimides.

In some embodiments, a modification that may render the nucleotide reactive involves the incorporation of a reactive group that is capable of reacting with another chemical group, e.g. a chemical group on a molecule or component to be conjugated to the functionalised oligonucleotide, via click chemistry. As used herein, the term "click chemistry," generally refers to reactions that are modular, wide in scope, give high yields, generate only inoffensive by-products, such as those that can be removed by nonchromatographic methods, and are stereospecific (but not necessarily enantioselective). See, e.g., Angew. Chem. Int. Ed., 2001, 40(11):2004-2021. In some cases, click chemistry can describe pairs of functional groups that can selectively react with each other in mild, aqueous conditions. Accordingly, click chemistry groups are suitable for the conjugation of additional functional groups to the oligonucleotides of the present method. Common click chemistry reactions include azide-alkyne cycloadditions, alkyne-nitrone cycloadditions, alkene-tetrazine reactions and alkene-tetrazole reactions. Accordingly, the functionalised nucleotide may comprise an azide group, an alkyne group, an alkene group, a nitrone group, a tetrazine group or a tetrazole group.

A specific example of click chemistry reaction can be the Huisgen 1,3-dipolar cycloaddition of an azide and an alkyne, i.e., Copper-catalysed reaction of an azide with an alkyne to form a 5-membered heteroatom ring called 1,2,3-triazole. The reaction can also be known as a Cu(I)-Catalyzed Azide-Alkyne Cycloaddition (CuAAC), a Cu(I) click chemistry or a Cu+ click chemistry. Catalyst for the click chemistry can be Cu(I) salts, or Cu(I) salts made in situ by reducing Cu(II) reagent to Cu(I) reagent with a reducing reagent (Pharm Res. 2008, 25(10): 2216-2230). Known Cu(ll) reagents for the click chemistry can include, but are not limited to, Cu(ll) (TBTA) complex and Cu(ll) (THPTA) complex. TBTA, which is tris-[(1-benzyl-1H-1,2,3-triazol-4-yl)methyl]amine, also known as tris-(benzyltriazolylmethyl)amine, can be a stabilizing ligand for Cu(I) salts. THPTA, which is tris-(hydroxypropyltriazolylmethyl)amine, can be another example of stabilizing agent for Cu(I). Other conditions can also be accomplished to construct the 1,2,3-triazole ring from an azide and an alkyne (e.g. a cycloalkyne, such as cyclooctyne or cyclononyne) using copper-free click chemistry, such as by the Strain-promoted Azide-Alkyne Click chemistry reaction (SPAAC, see, e.g., Chem. Commun., 2011, 47:6257-6259 and Nature, 2015, 519(7544):486-90).

In some embodiments, the functionalised nucleotide contains a reactive group in the in the sugar group, e.g. a modification at position 2 in the deoxyribose sugar, such as substituting the hydrogen with a fluoro, chloro, bromo or azide group. In some embodiments, the functionalised nucleotide is a 2'-Azido-dNTP, e.g. 2'-Azido-dATP.

In some embodiments, the functionalised nucleotide contains a reactive group in the nucleobase, particularly selected from an alkyne, alkenyl, thio or halogen group. As noted above, the functionalised nucleotide may comprise a pyrimidine comprising a substitution at the C5 position. In some embodiments, the alkyne group is ethyne, e.g. the functionalised nucleotide is an ethynyl-dNTP, such as 5'-Ethynyl-dUTP. Alternatively, the alkyne group may be a propynyl group, e.g. the functionalised nucleotide is a propynyl dNTP, such as 5'-Propynyl-dUTP. In some embodiments, the alkenyl group is vinyl (ethenyl), e.g. the functionalised nucleotide is a vinyl-dNTP, such as 5'-vinyl-dUTP. In some embodiments, the functionalised nucleotide is a thio-dNTP, such as 4'-thio-dTTP. In some embodiments, the halogen group is bromine, e.g. the functionalised nucleotide is a bromo-dNTP, such as 5'-Bromo-dUTP. The incorporation of halogen groups into functionalised oligonucleotides can be used to promote nucleophilic aromatic substitutions or UV mediated crosslinking, e.g. with proteins. Thus, in some embodiments, functionalised oligonucleotides produced by the present method may be further modified to comprise aromatic groups or may by conjugated to other molecules, e.g. proteins or peptides, via UV mediated crosslinking.

In some embodiments, a functionalised nucleotide may contain a group capable of interacting with another component, e.g. interacting via a non-covalent bond. For instance, the nucleotide may be modified to incorporate one part or component of a cognate binding pair, e.g. an affinity binding partner, e.g. biotin or a hapten, capable of binding to its binding partner, i.e. a cognate binding partner, e.g. streptavidin or an antibody. Such functionalised nucleotides may, for example, find utility in the production of functionalised oligonucleotides that may be immobilised on a solid support.

In some embodiments, the functionalised nucleotide contains a modification that renders the oligonucleotide containing the nucleotide resistant to degradation, e.g. chemical and/or enzymatic degradation (e.g. nuclease degradation). In some embodiments, the nucleotide contains a modification in the sugar group, e.g. a modification at position 2 in the deoxyribose sugar, such as substituting the hydrogen with a fluoro, chloro, O-methyl or O-ethyl group. Thus, in some embodiments, the functionalised nucleotide is a 2'-fluoro-dNTP, e.g. 2-fluoro-UTP. In some embodiments the functionalised nucleotide comprises an O-Me group, e.g. 2'-O-Methyl-ATP. In some embodiments, the nucleotide contains a modification in the phosphate group that forms the internucleotide linkage, such as substituting an oxygen with a sulfur, e.g. the nucleotide contains a phosphorothionate group. Thus, in some embodiments, the functionalised nucleotide is nucleotide thiotriphosphate, e.g. 2-deoxythymidine-5'-O-(1-thiotriphosphate), 2-deoxycytidine-5'-O-(1-thiotriphosphate), 2-deoxyuridine-5'-O-(1-thiotriphosphate), 2-deoxyadenosine-5'-O-(1-thiotriphosphate) or 2-deoxyguanosine-5'-O-(1-thiotriphosphate). In some embodiments, the functionalised nucleotide contains a modification in the nucleobase, such as an amino, methyl, ethyl or propynyl modification, e.g. 2-amino-dATP, 5-methyl-dCTP, C-5 propynyl-dCTP or C-5 propynyl-dUTP.

In some embodiments, the functionalised nucleotide contains a modification that affects the thermostability of the oligonucleotide. In some embodiments, the nucleotide comprises a locked ribose sugar, i.e. comprises an additional covalent bond between the 2' oxygen and the 4' carbon of the pentose ring. In some embodiments, the nucleotide is an LNA (locked nucleic acid) nucleotide, i.e. LNA-NTP such as LNA-ATP. The locked ribose conformation enhances base stacking and thus increases the melting temperature of oligonucleotides comprising LNA nucleotides.

Thus, in some embodiments, the functionalised nucleotides that may be used in the invention include: nucleotides comprising an (internalized) alkyne or azide group, fluorescently labelled nucleotides, nucleotides comprising a sterol group, nucleotides comprising a polyether group, nucleotides comprising a metal complex, nucleotides comprising a vinyl group, nucleotides comprising a thiol group, thionated nucleotides, nucleotides modified to have increased nuclease resistance, nucleotides comprising a chemical group capable of participating in click chemistry, nucleotides that affect (e.g. increase) the thermostability of the oligonucleotide (e.g. LNA-nucleotides) or a combination thereof. The incorporation of these functionalised nucleotides into the single stranded oligonucleotides provided by the method of the present invention can provide a range of useful functions. For example, single stranded oligonucleotides comprising fluorophores can be used as sequence specific fluorescent probes. The inclusion of thiolated nucleotides in a single stranded oligonucleotide allows the oligonucleotide to be labelled with thiol-reactive molecules, and to be used as a probe for molecular detection of such thiol-reactive molecules.

In some embodiments, the functionalised nucleotides that may be used in the invention do not include nucleotides comprising a digoxigenin group. Alternatively put, in some embodiments, the functionalised nucleotides are not digoxigenin labelled nucleotides. In particular, in some embodiments the functionalised nucleotides are not digoxigenin-11-dUTP.

In a preferred embodiment, the functionalised dNTPs are nucleotides comprising an alkyne group or a vinyl group, e.g. a modified nucleobase containing an alkyne (e.g. ethynyl) group or vinyl group, e.g. a nucleotide comprising a pyrimidine with an alkyne or vinyl group at position C5. In another preferred embodiment, the functionalised dNTPs are nucleotides comprising an azide group, e.g. comprising a modified sugar containing an azide group, e.g. a nucleotide comprising an azide group at position 2 of the deoxyribose sugar.

The reaction mixture for the RCA reaction must contain a combination of components capable of generating an RCA product from the template circular DNA molecule. For instance, the nucleotides present in the reaction mixture (e.g. the mixture of conventional and functionalised nucleotides) must be capable of hybridising to their respective nucleotides in the circular DNA template to permit rolling circle amplification. The relative amounts of functionalised and conventional nucleotides present in the reaction mixture may vary depending on the identity of the functionalised nucleotide and the polymerase. Moreover, the relative amount of each nucleotide present in the reaction mixture may be used to control the incorporation of functionalised nucleotides into the RCA product. For instance, increasing the concentration of the functionalised nucleotides (or decreasing the proportion of conventional nucleotides) may result in a greater proportion of functionalised nucleotides in the RCA product (e.g. when the functionalised nucleotide is used in combination with its equivalent conventional nucleotide). Conversely, decreasing the concentration of the functionalised nucleotides (or increasing the proportion of conventional nucleotides) may result in a lower proportion of functionalised nucleotides in the RCA product.

Thus, the functionalised nucleotides may be used in addition to, or entirely in place of, the conventional nucleotides which hybridise to the same DNA base (nucleotide) in the template DNA. In some embodiments, the reaction mixture may contain only one type of functionalised nucleotide. In some embodiments, a combination of different functionalised nucleotides may be used in the same reaction. In some embodiments, all of the functionalised nucleotides in the reaction mixture contain the same type of functional group, e.g. alkyne group. In some embodiments, the functionalised nucleotides in the reaction mixture contain the different types of functional group. By way of example, the different types of functionalised nucleotides may be capable of hybridising to the same DNA base (nucleotide), such as a dATP nucleotide functionalised with a fluorophore and a second dATP nucleotide functionalised with a sterol group. In another representative example, the different types of functionalised nucleotides may be capable of hybridising to different DNA bases (nucleotides), such as a dATP nucleotide functionalised with a fluorophore and a dTTP nucleotide functionalised with a sterol group (or a different fluorophore to the fluorophore on the dATP nucleotide). Thus, any combination of functionalised and conventional nucleotides may be used in the invention. In a preferred embodiment, the RCA reaction mixture contains only one type of functionalised nucleotide.

The amount of functionalised nucleotides present in the reaction mixture can be measured as a relative percentage of the total nucleotides capable of hybridising to a particular DNA base (nucleotide). Alternatively, this value can be considered as the percentage to which the functionalised nucleotide has replaced the corresponding conventional nucleotide. For example, using equal amounts of conventional dATP and a dATP modified with a fluorophore could be expressed as 50% of the total dATP nucleotides being modified (functionalised), or 50% replacement of the conventional dATP nucleotides with functionalised dATP nucleotides.

The relative amount of functionalised nucleotides in the RCA reaction mixture can be varied in order to control the frequency of functionalised nucleotides in the final single stranded oligonucleotides. In some embodiments, the functionalised nucleotides may represent up to about 5% of the total nucleotides capable of hybridising to a particular DNA base (nucleotide), for example about 1%, 2%, 3%, 4% or 5%. Alternatively, in some embodiments the functionalised nucleotide may represent a higher proportion, such as 25%, 50%, 75% or 100% of the total nucleotides capable of binding to a particular DNA base (nucleotide).

The relative amount of functionalised nucleotides present may be varied for numerous reasons. For instance, some functionalised nucleotides, such as dATP modified with Cy3 are not available commercially at high concentrations. Moreover, as shown in the Examples, the inventors have determined that the inclusion of functionalised nucleotides may impact the yield of functionalised single stranded oligonucleotides produced by the invention, e.g. the use of high relative amounts of the functionalised nucleotide can inhibit the activity of the DNA polymerase responsible for the RCA reaction (e.g. reduce the efficiency at which the RCA product is synthesised), or the cleavage enzyme responsible for cleaving the RCA product and releasing the single stranded functionalised oligonucleotides (e.g. reduce the efficiency at which the RCA product is cleaved).

Notably however, the inventors have surprisingly determined that high yields of functionalised single stranded oligonucleotides may be achieved even when using high relative amounts of functionalised oligonucleotides, e.g. up to about 75%, such as up to about 70%, 65%, 60%, 55% or 50%. In some embodiments, it may be possible to use more than 75% of the functionalised oligonucleotides, such as about 80%, 85%, 90%, 95% or 100%. In particular, the inventors have unexpectedly found that functionalised nucleotides containing alkyne or vinyl groups in the nucleobase are particularly useful in the invention as they may be incorporated into the RCA product efficiently. Moreover, the RCA product could readily be cleaved to yield the functionalised single stranded oligonucleotides, although as discussed below, in some embodiments a higher amount of cleavage enzyme may be required relative to the amount needed to cleavage an equivalent RCA product containing only conventional nucleotides.

Similarly, the inventors have found that functionalised nucleotides containing O-methyl groups in the deoxyribose sugar may completely substitute a conventional nucleotide in the RCA reaction and still yield RCA products. Furthermore, the inventors surprisingly determined that incorporation of these nucleotides into the RCA products does not affect formation of the cleavage domains (e.g. hairpin cleavage domains) or their enzymatic cleavage (e.g. with restriction endonucleases).

Accordingly, the relative amount of functionalised nucleotide that is present in the reaction mixture may be adjusted to optimise the yield of the desired functionalised single stranded oligonucleotides or to optimise the generation of the RCA product. Such modifications are within the purview of the skilled person based on the methods described in the Examples below. Thus, in some embodiments, the relative amount of functionalised nucleotides in the reaction mixture may be about 1-5%, 1-10%, 1-25%, 5-25%, 10-25%, 25-50%, 25-75%, 25-100%, 50-75%, 50-100%, or 75-100%.

The term "release" is used in the present context to refer to cleaving the RCA product at the cleavage domains bordering the oligonucleotide sequences so as to detach or separate the functionalised oligonucleotides from the cleavage domains. It is desirable that the release of a given functionalised oligonucleotide will involve cleavage at both cleavage domains bordering the oligonucleotide sequence.

It is not necessary for cleavage to occur at all of the cleavage domains in the RCA product in order to generate the functionalised single stranded oligonucleotides. As noted above, incorporation of functionalised nucleotides in the RCA product, particularly in the cleavage domains, may reduce the efficiency of the cleavage step. Nevertheless, cleavage of a portion of cleavage domains will result in the release of a portion of functionalised single stranded oligonucleotides. Thus, in some embodiments, the step of cleaving the RCA product results in cleavage of at least about 30% of the cleavage domains in the RCA product, e.g. at least about 35%, 40%, 45%, 50%, 60%, 70% or 80%. In some embodiments, the step of cleaving the RCA product results in cleavage of at least about 90% of the cleavage domains in the RCA product, e.g. 95% or more.

Alternatively viewed, in some embodiments, the step of cleaving the RCA product results in the release of at least about 30% of the functionalised single stranded oligonucleotides contained in the RCA product, e.g. at least about 35%, 40%, 45%, 50%, 60%, 70% or 80%. In some embodiments, the step of cleaving the RCA product results in the release of at least about 90% of the functionalised single stranded oligonucleotides contained in the RCA product, e.g. 95% or more.

Once the single stranded functionalised oligonucleotides have been released they are isolated, separated or purified from the cleavage reaction mixture (e.g. reaction components and/or degradation products such as cleavage domains, uncleaved RCA products etc.).

Thus, the method of the present invention comprises a step of isolating, separating or purifying the functionalised single stranded oligonucleotides. This isolation, separation or purification may be done by any suitable method known in the art.

In some embodiments, following the isolation, separation or purification step the functionalised single stranded oligonucleotides are preferably substantially free of any contaminating components derived from the materials or component used in the isolation procedure or in their preparation (e.g. reaction components and/or degradation products such as cleavage domains, uncleaved RCA products etc.). In some embodiments, the functionalised single stranded oligonucleotides are purified to a degree of purity of more than about 50 or 60%, e.g. more than about 70, 80 or 90%, such as more than about 95 or 99% purity as assessed w/w (dry weight). Such purity levels may include degradation products of the functionalised single stranded oligonucleotides.

In some embodiments, it may be useful to prepare enriched preparations of the functionalised single stranded oligonucleotides which have lower purity, e.g. contain less than about 50% of the functionalised single stranded oligonucleotides of interest, e.g. less than about 40 or 30%.

As discussed above, the invention may result in a mixture or plurality (e.g. a library) of functionalised single stranded oligonucleotides. Thus, in some embodiments, it may be desirable to further separate the functionalised single stranded oligonucleotides, e.g. by size, to obtain specific functionalised single stranded oligonucleotides (i.e. to isolate specific functionalised single stranded oligonucleotides) or to generate sub-groups or sub-libraries of functionalised single stranded oligonucleotides. Any suitable means for separating the mixtures of functionalised single stranded oligonucleotides to isolate the specific functionalised single stranded oligonucleotides or sub-groups or sub-libraries of functionalised single stranded oligonucleotides may be employed.

Thus, in some embodiments, the method comprises a further step of separating functionalised single stranded oligonucleotides from a mixture (e.g. library) of functionalised single stranded oligonucleotides obtained by the method described above, to isolate a specific functionalised single stranded oligonucleotide or a sub-group of functionalised single stranded oligonucleotides.

For example, the products of the cleavage reaction may be separated by size using gel electrophoresis using an agarose gel or a polyacrylamide gel. The desired functionalised oligonucleotides can then be isolated from the gel and purified further, if necessary, according to methods known in the art. Other methods for purifying, isolating or separating the functionalised oligonucleotides utilise chromatography (e.g. HPLC, size-exclusion, ionexchange, affinity, hydrophobic interaction, reverse-phase) or capillary electrophoresis.

As mentioned above, the functionalised oligonucleotides produced by the method described above may contain a reactive group that is capable of reacting with another chemical group, e.g. a chemical group on a molecule or component to be conjugated to the functionalised oligonucleotide, e.g. via click chemistry. For instance, conjugating additional molecules or components (which themselves may comprise or be viewed as functional groups) to the single stranded oligonucleotides may be particularly useful for incorporating large or bulky groups, such as groups that may inhibit or lower the efficiency of the present method if present in the functionalised nucleotides used in the RCA reaction. Thus, for example, functionalised oligonucleotides may be produced that contain molecules or components that could not be incorporated by a polymerase directly during the RCA reaction, or would only be incorporated at low efficiencies or yields. Additionally or alternatively, subjecting the functionalised oligonucleotides obtained by the method to a further conjugation step may increase the diversity of the structures present in a functionalised oligonucleotide library.

Accordingly, in some embodiments, the method further comprises a step of conjugating a molecule or component to the functionalised oligonucleotide(s) via a functional (e.g. reactive) group in the oligonucleotide, such as via click chemistry. In some preferred embodiments, the molecule or component is conjugated to the functionalised oligonucleotide via an alkyne, vinyl or azide group (i.e. an alkyne, vinyl or azide group in a functionalised nucleotide incorporated into the RCA product in the method defined herein).

The term "conjugation" in the context of the present invention with respect to linking or joining a molecule or component to a functionalised oligonucleotide (e.g. an alkyne, vinyl or azide group in said functionalised oligonucleotide) refers to joining said molecule or component to said oligonucleotide via a covalent bond. In particular, this conjugation may occur via a click chemistry reaction.

An example of a specific click chemistry reaction that may be used to conjugate additional molecules or components to single stranded functionalised oligonucleotides comprising one or more alkyne groups is the azide-alkyne cycloaddition. In order to achieve the desired conjugation, the oligonucleotide comprising the alkyne group may be incubated for a suitable period of time with a molecule or component (e.g. a label such as a fluorophore) containing an azide group. The azide-alkyne cycloaddition reaction commonly uses a copper catalyst, particularly a copper(I) catalyst. In some embodiments, the oligonucleotide and the azide-containing molecule or component may be incubated in the presence of copper sulfate. A reducing agent may also be used to generate the active copper(I) catalyst. The reducing agent may be, for example, sodium ascorbate.

A further representative example for conjugating additional molecules or components to single stranded functionalised oligonucleotides comprising one or more vinyl groups may utilise the alkene-tetrazine reaction. This reaction has the advantage of being copper free. Moreover, it is entirely orthogonal to the aforementioned alkyne-azide click chemistry reaction. Accordingly, a single stranded functionalised oligonucleotide comprising both an alkyne group and a vinyl group could participate in two click chemistry reactions independently to conjugate two different additional molecules or components to the same oligonucleotide.

Thus, in some embodiments, the invention may be seen as providing a two-step method for producing functionalised single stranded oligonucleotides comprising a first step of incorporating functionalised nucleotides (e.g. comprising reactive groups, such as groups capable of participating in click chemistry reactions, e.g. alkyne, vinyl or azide groups) into an oligonucleotide using the method described herein, and a second step of conjugating additional molecules or components to the single stranded oligonucleotides via the functional groups in the functionalised nucleotides.

It will be evident that any desirable molecules or components (i.e. entities) may be conjugated to the functional groups in the single stranded oligonucleotides produced by the present method. Such molecules or components simply require the presence of a group (e.g. reactive group) capable of reacting with a functional group in the oligonucleotide to form a covalent bond. In some embodiments, the molecule or component may be a nucleic acid molecule, protein, peptide, small-molecule organic compound (e.g. a sterol, such as cholesterol), fluorophore, metal-ligand complex, polysaccharide, nanoparticle, nanotube, polymer, cell, organelle, vesicle, virus, virus-like particle or any combination of these.

The cell may be a prokaryotic or eukaryotic cell. In some embodiments the cell is a prokaryotic cell, e.g. a bacterial cell.

In some embodiments, the functionalised oligonucleotide may be conjugated or fused to a compound or molecule which has a therapeutic or prophylactic effect, e.g. an antibiotic, antiviral, vaccine, antitumour agent, e.g. a radioactive compound or isotope, cytokine, toxin, oligonucleotide, nucleic acid encoding gene or nucleic acid vaccine.

In some embodiments, the functionalised oligonucleotide (e.g. an aptamer) may be conjugated or fused to a label, e.g. a radiolabel, a fluorescent label, luminescent label, a chromophore label as well as to substances and enzymes which generate a detectable substrate, e.g. horse radish peroxidase, luciferase or alkaline phosphatase. This detection may be applied in numerous assays where antibodies are conventionally used, including Western blotting/immunoblotting, histochemistry, enzyme-linked immunosorbent assay (ELISA), or flow cytometry (FACS) formats. Labels for magnetic resonance imaging, positron emission tomography probes and boron 10 for neutron capture therapy may also be conjugated to the functionalised oligonucleotides described herein.

In some embodiments, the molecule or component may be selected from the group consisting of: a fluorophore, a sterol (e.g. cholesterol), a polyether, a metal complex, a thiol containing molecule, a molecule containing a group providing increased nuclease resistance and a molecule containing a group capable of participating in a click chemistry reaction.

It will be evident that the molecule or component conjugated to the functionalised oligonucleotide may interact with other molecules and such interactions may be covalent or non-covalent interactions. For instance, a peptide conjugated to the oligonucleotide may interact with its cognate binding partner, such as an antibody, non-covalently. In a further example, a molecule containing a group capable of participating in a click chemistry reaction may be conjugated to another molecule or component as defined above via reaction with a reactive group of said molecule or component to form a covalent complex.

The circular DNA molecule provided in step (a) of the present method may be produced by any suitable means and a variety of means are well-known in the art. Accordingly, the method of the present invention may comprise additional steps before step (a) of providing a circular DNA molecule.

For instance, the process of producing the circular DNA molecule may comprise a step of designing a sequence comprising one or more desired oligonucleotide sequences bordered by cleavage domains (termed a "pseudogene" herein) *in silico.* Thus, the method may utilise a computer-implemented method of designing the pseudogene and such methods are described in the art, e.g. Ducani et al., 2013, *supra.*

The term "pseudogene" as used herein refers to a nucleotide sequence comprising one or more desired oligonucleotide sequences, bordered by cleavage domains. Alternatively, this sequence may be referred to herein as the "nucleic acid construct".

The pseudogene sequence designed *in silico* can be produced (e.g. synthesised) using commercially available gene synthesis methods, or any other suitable means known in the art, e.g. assembly PCR. Thus, the method may comprise a step of producing the pseudogene.

The pseudogene may be produced as a single stranded or double stranded molecule. The pseudogene may circularised using any suitable means known in the art to provide the circular DNA molecule of step (a). For instance, a double stranded molecule may be ligated using a ligase enzyme, as described below. It will be understood in this regard that at least one of the 5' ends of the pseudogene is phosphorylated to enable ligation to take place. In embodiments where the step of producing the pseudogene results in a DNA molecule in which the 5' ends are not phosphorylated, the method may comprise a further step of phosphorylating the 5' end(s) of the pseudogene, e.g. using a kinase enzyme, such as T4 polynucleotide kinase.

Similarly, where the pseudogene is provided as a single stranded molecule, it may be circularised using an appropriate ligase enzyme capable of catalysing intramolecular ligation of single stranded oligonucleotide molecules, such as CircLigase. Again, the method may comprise a further step of phosphorylating the 5' end(s) of the pseudogene, e.g. using a kinase enzyme, such as T4 polynucleotide kinase, to facilitate the ligation reaction.

In some embodiments it may be advantageous to insert the pseudogene into a plasmid, which can be replicated in bacteria, such as *E.coli.* Suitable plasmid sequences are well-known in the art. This allows the sequence of the pseudogene to be checked (e.g. by sequencing) and for any errors in the sequence to be corrected, e.g. via iterations of sequencing and mutagenesis using any suitable methods.

The insertion of the pseudogene into a plasmid also facilitates the generation of a significant number of copies of the circular DNA molecule. For instance, a single bacterial colony containing a plasmid comprising the sequence-verified pseudogene may be grown to amplify the plasmid, which may be subsequently purified from the bacteria using any suitable means known in the art.

The pseudogene sequence is then excised from the plasmid, e.g. using restriction enzymes as described above. The excised linear pseudogene sequence may be purified using PAGE and gel extraction, or other suitable methods. The purified linear pseudogene sequence can then be re-circularised using a suitable ligase enzyme, such as T4 ligase, to form the circular DNA molecule to be provided in step (a) of the present method.

In addition to amplification, the process of transfecting the pseudogene containing plasmid into bacteria also allows a bacterial glycerol stock to be produced. Bacteria comprising the desired pseudogene plasmid can be prepared in glycerol, frozen and stored stably for long periods of time.

Accordingly, in some embodiments step (a) of the present method comprises:
(i) cloning into a DNA plasmid a linear DNA molecule comprising the oligonucleotide sequence bordered by cleavage domains;
(ii) amplifying said plasmid;
(iii) excising part of the plasmid containing the DNA molecule comprising the oligonucleotide sequence bordered by cleavage domains; and
(iv) circularising the part of the plasmid obtained in step (iii).

In some embodiments, step (ii) comprises transfecting said DNA plasmid into bacteria and growing the bacteria.

In some embodiments, the linear DNA molecule comprising the oligonucleotide sequence bordered by cleavage domains further comprises a 5' end region and a 3' end region each comprising a cleavage domain and wherein step (iii) comprises cleaving the cleavage domains in the end regions with a cleavage enzyme.

Any suitable cleavage enzyme as defined herein may be used to excise the pseudogene from the plasmid. In some embodiments, the cleavage enzyme is BsmBl or Bsal or an isoschizomer thereof.

In some particular embodiments of the invention, the method uses a phi29 DNA polymerase or a derivative thereof and a functionalised nucleotide comprising a modified nucleobase. In some embodiments, the functionalised nucleotide comprises a reactive group in the nucleobase, such as a reactive group capable of participating in a click chemistry reaction. In some embodiments, the reactive group in the nucleobase is an alkyne or a vinyl group as defined above. In some embodiments, the relative amount of functionalised nucleotide in the RCA mixture is about 25-100%, e.g. 25-75%, 50-100% or 75-100% or any value within these ranges. The cleavage domains are capable of forming hairpin structures in the RCA product as defined above.

In a further aspect, the present invention provides a kit for use in the method described herein comprising:
(i) a circular DNA molecule comprising an oligonucleotide sequence bordered by cleavage domains, wherein the cleavage domains comprise or consist of a sequence capable of forming a hairpin structure and wherein the double-stranded portion of the hairpin structure comprises a sequence that is recognised by a cleavage enzyme;
(ii) functionalised dNTPs (e.g. as defined herein); and
(iii) one or more cleavage enzymes that cleave the cleavage domains of (i).

In some embodiments, the kit may comprise a DNA polymerase enzyme capable of performing rolling circle amplification, i.e. comprising at least some strand displacement activity, as defined herein. For instance, the kit may comprise a phi29 polymerase or derivative thereof, or a Bst DNA polymerase or derivative thereof.

In some embodiments, the kit may comprise one or more nickases. For instance, the kit may comprise Nb.BsrDl, Nt.BspQl or a combination thereof.

In some embodiments, the kit may comprise one or more single stranded binding protein as defined herein. For instance, the kit may comprise E. coli single stranded DNA binding protein, gene 32 protein of T4 phage, or a combination thereof.

In some embodiments, the kit may comprise one or more molecules or components to be conjugated to a functionalised single stranded oligonucleotide as defined above.

The circular DNA molecule, cleavage enzymes and functionalised dNTPs of the kit are as described above.

In some embodiments, at least 5% of the nucleotide residues of the single stranded functionalised oligonucleotide produced by the method are functionalised nucleotides, i.e. nucleotides containing a functional group as defined herein. In some embodiments, at least 10%, 15%, 20%, 25%, 30%, 35%, 40%, 45%, 50% or more of the nucleotide residues of the single stranded functionalised oligonucleotide produced by the method are functionalised nucleotides. Thus, in some embodiments, about 5-100% of the nucleotide residues of the single stranded functionalised oligonucleotide produced by the method are functionalised nucleotides, e.g. about 10-95%, 15-90%, 20-85%, 25-80%, 30-75%, 35-70%, 40-65% or 45-55% of the nucleotide residues of the single stranded functionalised oligonucleotide produced by the method are functionalised nucleotides.

In some embodiments, the single stranded functionalised oligonucleotide produced by the method may contain at least one internal functionalised nucleotide, i.e. a functionalised nucleotide that is not at the 5' or 3' end of the oligonucleotide. In some embodiments, the single stranded functionalised oligonucleotide produced by the method may contain 2, 3, 4, 5, 6, 7, 8, 9, 10 or more internal functionalised nucleotides, e.g. 15, 20, 25, 30, 35, 40, 45, 50 or more internal functionalised nucleotides.

The single stranded functionalised oligonucleotide produced by the method may contain any one or more of the functionalised nucleotides defined herein. The single stranded functionalised oligonucleotide produced by the method may comprise functionalised nucleotides containing a functional group selected from an alkyne group, an alkene group (e.g. a vinyl group), an azide group, a halogen group, an O-methyl group, a locked ribose sugar or a combination thereof.

Thus, a single stranded functionalised oligonucleotide may be obtained by the method described herein, wherein;
(i) the oligonucleotide contains at least 50 nucleotides; and
(ii) at least 5% of the nucleotide residues contain a functional group selected from an alkyne group, an alkene group (e.g. vinyl), an azide group, a halogen group, an O-methyl group, a locked ribose sugar or a combination thereof, wherein the preferred positions of the functional groups within the functionalised nucleotide residues are as defined above.

A library comprising a plurality of single stranded functionalised oligonucleotides may be obtained by the method as described herein. The library of a plurality of single stranded functionalised oligonucleotides may include one or more single stranded functionalised oligonucleotides as defined above.

In yet a further aspect, the present invention provides a method as defined herein being a method for producing a pool of single stranded functionalised oligonucleotides for use in single molecule fluorescence in situ hybridisation (smFISH), wherein the functionalised nucleotides are fluorescently labelled nucleotides and wherein each single stranded functionalised oligonucleotide in the pool contains about 15-30, preferably about 20-25 nucleotides. In preferred embodiments, the functionalised nucleotides are labelled with the same fluorescent molecule.

As an alternative to the use of directly fluorescently labelled functionalised nucleotides, the method may involve the use of nucleotides comprising a chemical group capable of participating in click chemistry, to which a fluorescent label can subsequently be conjugated. Suitable fluorescent labels are well known in the art and are defined above.

Accordingly, in yet a further aspect, the present invention provides a method as defined herein being a method for producing a pool of single stranded functionalised oligonucleotides for use in single molecule fluorescence in situ hybridisation (smFISH), wherein the functionalised nucleotides are nucleotides comprising a chemical group capable of participating in click chemistry, and wherein the method further comprises a step of conjugating a fluorescent label to at least one functionalised nucleotide in each functionalised oligonucleotide via click chemistry, and wherein each single stranded functionalised oligonucleotide in the pool contains about 15-30, preferably about 20-25, nucleotides. Nucleotides comprising a chemical group capable of participating in click chemistry are defined above, and include nucleotides comprising an azide group, an alkyne group, an alkene group, a nitrone group, a tetrazine group or a tetrazole group, or a combination thereof.

In the smFISH technique, the single stranded functionalised oligonucleotides act as hybridisation probes to identify the presence and location of nucleic acid molecules which comprise a specific target sequence to be detected. Accordingly, once the pool of single stranded functionalised oligonucleotides for use in single molecule fluorescence in situ hybridisation (smFISH) has been produced by the method disclosed herein, the functionalised oligonucleotides may be hybridised to a nucleic acid molecule comprising a target sequence to be detected in an smFISH method.

Where the single stranded functionalised oligonucleotides produced by the method comprise functionalised nucleotides comprising a chemical group capable of participating in click chemistry, the step of conjugating a fluorescent label to the functionalised nucleotides via click chemistry may be carried out before or after the functionalised oligonucleotides are hybridised to a nucleic acid molecule comprising a target sequence.

For instance, the functionalised oligonucleotides comprising functionalised nucleotides comprising a chemical group capable of participating in click chemistry may be subjected to a step of conjugating a fluorescent label to at least one functionalised nucleotide in each functionalised oligonucleotide via click chemistry, and may then be hybridised to a nucleic acid molecule comprising a target sequence, once the fluorescent labels have been conjugated. Alternatively, the functionalised oligonucleotides comprising functionalised nucleotides comprising a chemical group capable of participating in click chemistry may first be hybridised to a nucleic acid molecule comprising a target sequence, and may then be subjected to a step of conjugating a fluorescent label to at least one functionalised nucleotide in each functionalised oligonucleotide via click chemistry, once hybridised to the target sequence.

Accordingly, in a yet further aspect, the present invention provides a method as defined herein being a method for producing a pool of single stranded functionalised oligonucleotides for use in single molecule fluorescence in situ hybridisation (smFISH), wherein the functionalised nucleotides are nucleotides comprising a chemical group capable of participating in click chemistry, and wherein the method further comprises a step of conjugating a fluorescent label to at least one functionalised nucleotide in each functionalised oligonucleotide via click chemistry, and wherein each single stranded functionalised oligonucleotide in the pool contains about 15-30, preferably about 20-25, nucleotides, and wherein the step of conjugating a fluorescent label to at least one functionalised nucleotide in each functionalised oligonucleotide via click chemistry is carried out before the functionalised oligonucleotides are hybridised to a nucleic acid molecule comprising a target sequence.

Similarly, the present invention also provides a method as defined herein being a method for producing a pool of single stranded functionalised oligonucleotides for use in single molecule fluorescence in situ hybridisation (smFISH), wherein the functionalised nucleotides are nucleotides comprising a chemical group capable of participating in click chemistry, and wherein the method further comprises a step of conjugating a fluorescent label to at least one functionalised nucleotide in each functionalised oligonucleotide via click chemistry, and wherein each single stranded functionalised oligonucleotide in the pool contains about 15-30, preferably about 20-25, nucleotides, and wherein the step of conjugating a fluorescent label to at least one functionalised nucleotide in each functionalised oligonucleotide via click chemistry is carried out after the functionalised oligonucleotides are hybridised to a nucleic acid molecule comprising a target sequence.

A "pool" of single stranded functionalised oligonucleotides refers to a plurality of oligonucleotides with different sequences, i.e. sequences that hybridise to different (e.g. nonoverlapping) sequences within the target (the molecule to be detected). In some embodiments, the pool contains at least about 20 oligonucleotides, e.g. about 22, 25, 27, 30, 35, 40, 45, 50, 60, 70, 80, 90 or 100 oligonucleotides. For instance, in some embodiments, the pool contains about 30-96 oligonucleotides, e.g. about 36, 48, 60, 72, 84 or 96 oligonucleotides.

Alternatively viewed, in some embodiments the pseudogene contains at least about 20 oligonucleotide sequences bordered by cleavage domains, e.g. about 22, 25, 27, 30, 35, 40, 45, 50, 60, 70, 80, 90 or 100 oligonucleotide sequences bordered by cleavage domains. For instance, in some embodiments, the pseudogene contains about 30-96 oligonucleotide sequences bordered by cleavage domains, e.g. about 36, 48, 60, 72, 84 or 96 oligonucleotide sequences bordered by cleavage domains.

The invention will now be described in more detail in the following non-limiting Examples with reference to the following drawings:

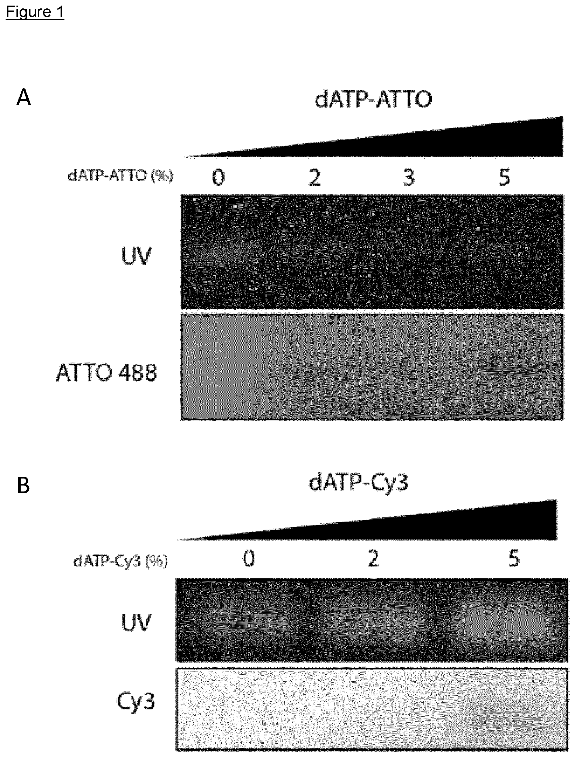

Figure 1 shows photographs of agarose gels visualised using UV light following ethidium bromide staining (top), and fluorescent imaging using wavelengths corresponding to the emission wavelengths of the fluorophores (bottom). The agarose gels show functionalised single stranded oligonucleotide products of the method of the invention (containing 378 nucleotides) comprising fluorophores ATTO-488 (A) or Cy3 (B).

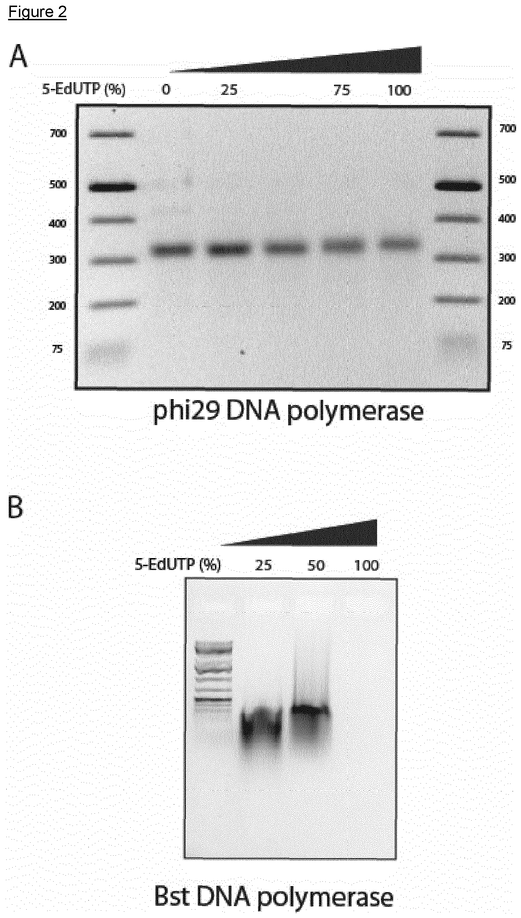

Figure 2 shows the negative image of a photograph of an agarose gel visualised using UV light following ethidium bromide staining. The agarose gel shows functionalised single stranded oligonucleotide products of the method of the invention (containing 420 nucleotides) comprising 5-ethnyl-dUTP (5-EdUTP) produced using various relative amounts of 5-EdUTP/dTTP nucleotides, i.e. 25%, 50%, 75% and 100% and phi29 DNA polymerase (A) or Bst DNA polymerase (B).

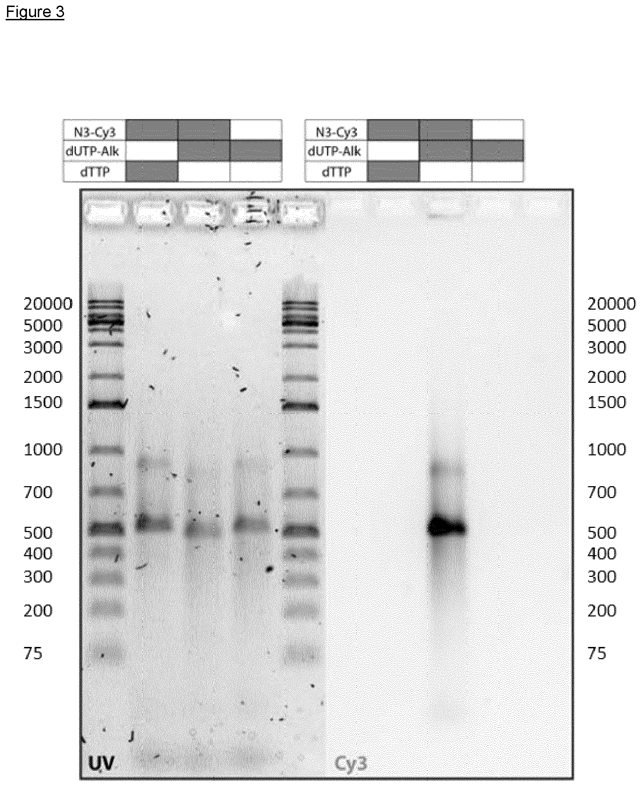

Figure 3 shows a photograph of an agarose gel visualised using UV light following ethidium bromide staining (left), and fluorescent imaging using wavelengths corresponding to the emission wavelengths of the Cy3 fluorophores (right). The agarose gels show single stranded oligonucleotide products of the method of the invention (containing 420 nucleotides) comprising 5-ethnyl-dUTP (5-EdUTP) or conventional dTTP that were subsequently reacted with Cy3 fluorophore-azide molecule (N3-Cy3). The shaded boxes denote the presence of the indicated species.

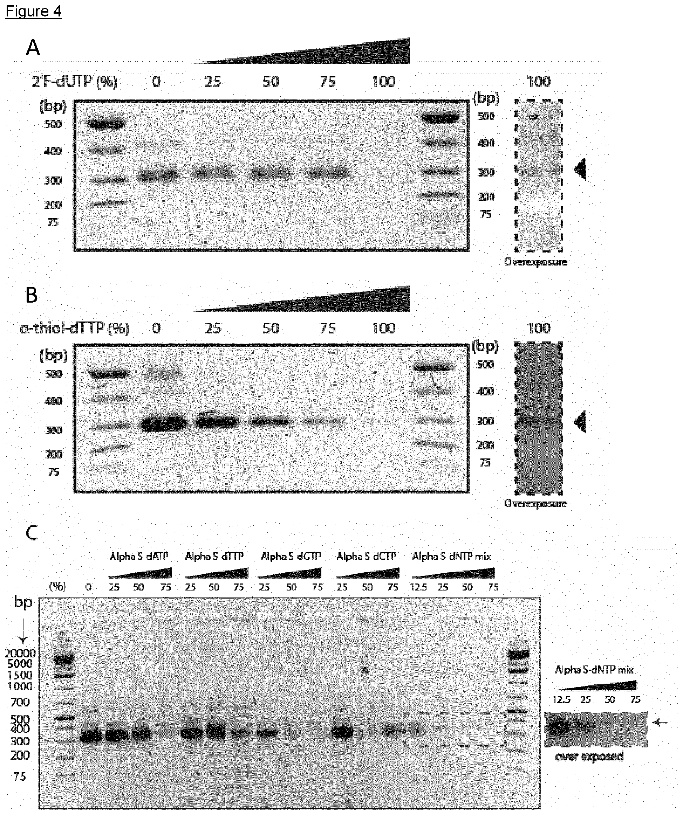

Figure 4 shows negative images of photographs of agarose gels visualised using UV light following ethidium bromide staining. The agarose gels show functionalised single stranded oligonucleotide products of the method of the invention (containing 420 nucleotides) comprising: (A) 2'-Fluoro-2'-deoxyuridine-5'-triphosphate (2'F-dUTP); (B) 2'-Deoxythymidine-5'-O-(1-Thiotriphosphate) (α-thiol-dTTP); and (C) 2-dNTP Alpha S nucleotides (Alpha S-dATP, Alpha S-dTTP, Alpha S-dCTP, Alpha S-dTTP, and an Alpha S-dNTP mixture), produced using various relative amounts of the functionalised nucleotides. The right panels in A and B show the lane corresponding to 100% functionalised nucleotides overexposed to show a band corresponding to the RCA product. The right panel in C show the lanes corresponding to Alpha S-dNTP mixture overexposed to show bands corresponding to the RCA product.

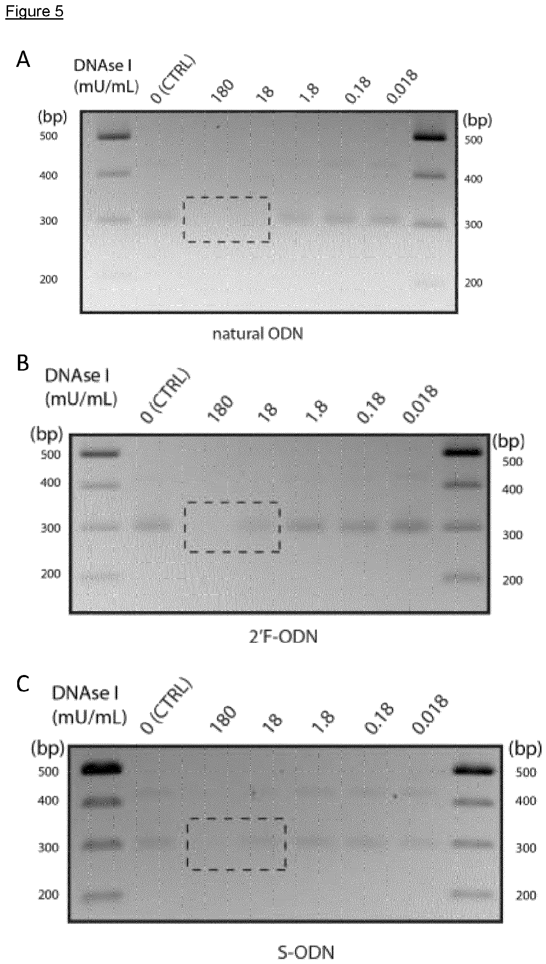

Figure 5 shows negative images of photographs of agarose gels visualised using UV light following ethidium bromide staining. The agarose gels show oligonucleotides produced by the method of the invention subjected to various concentrations of DNase I, wherein: (A) shows the reaction products of an oligonucleotide containing only conventional nucleotides (natural ODN); (B) shows the reaction products of an oligonucleotide containing 2'-Fluoro-2'-deoxyuridine-5'-triphosphate functionalised nucleotides (2'F-dUTP); and (C) shows the reaction products of an oligonucleotide containing 2'-Deoxythymidine-5'-O-(1-Thiotriphosphate) (S-ODN).

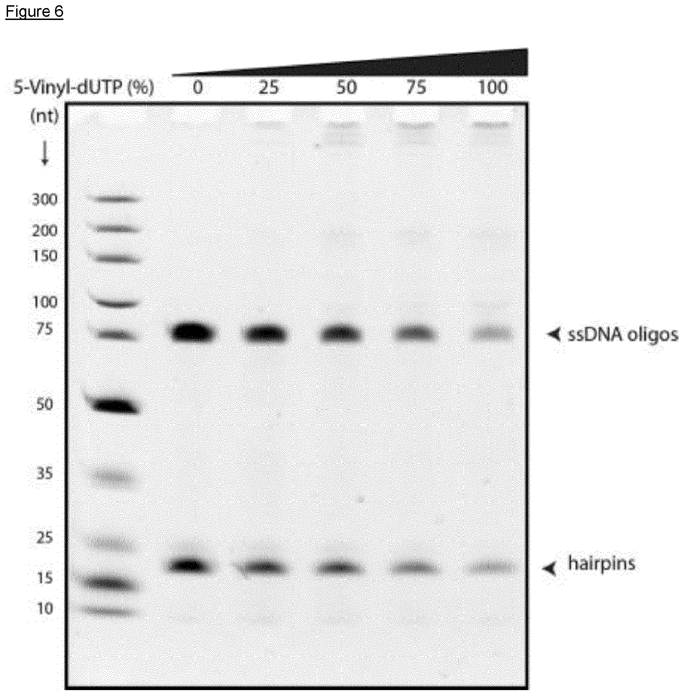

Figure 6 shows a negative image of a photograph of a denaturing PAGE gel visualised using UV light following SybrGold staining. The PAGE gel shows functionalised single stranded oligonucleotide products of the method of the invention comprising 5-Vinyl-2'-deoxyuridine-5'-triphosphate (5-Vinyl-dUTP) produced using various relative amounts of 5-Vinyl-dUTP nucleotides, i.e. 25%, 50%, 75% and 100%.

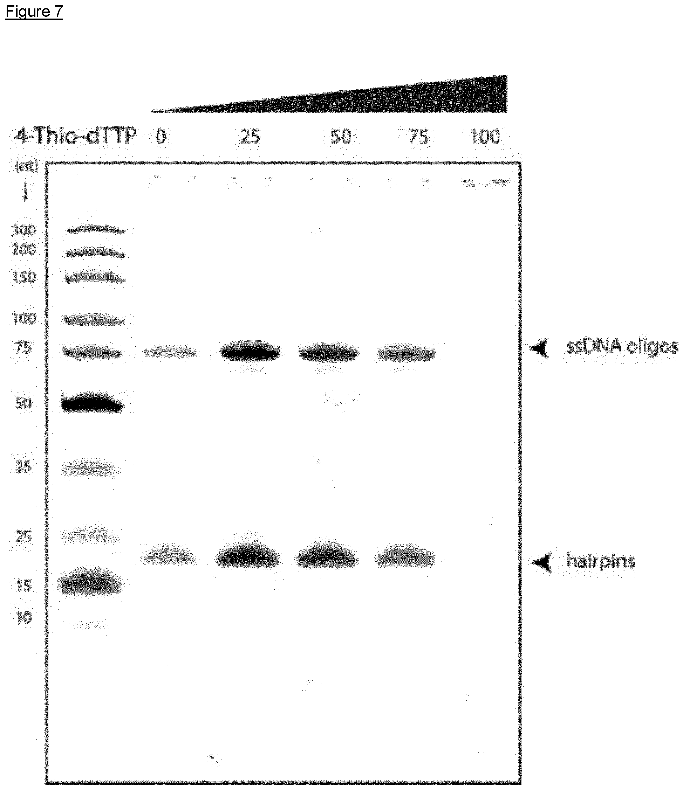

Figure 7 shows a negative image of a photograph of a denaturing PAGE gel visualised using UV light following SybrGold staining. The PAGE gel shows functionalised single stranded oligonucleotide products of the method of the invention comprising 4-Thiothymidine-5'-Triphosphate (4-Thio-dTTP) produced using various relative amounts of 5-Vinyl-dUTP nucleotides, i.e. 25%, 50%, 75% and 100%.

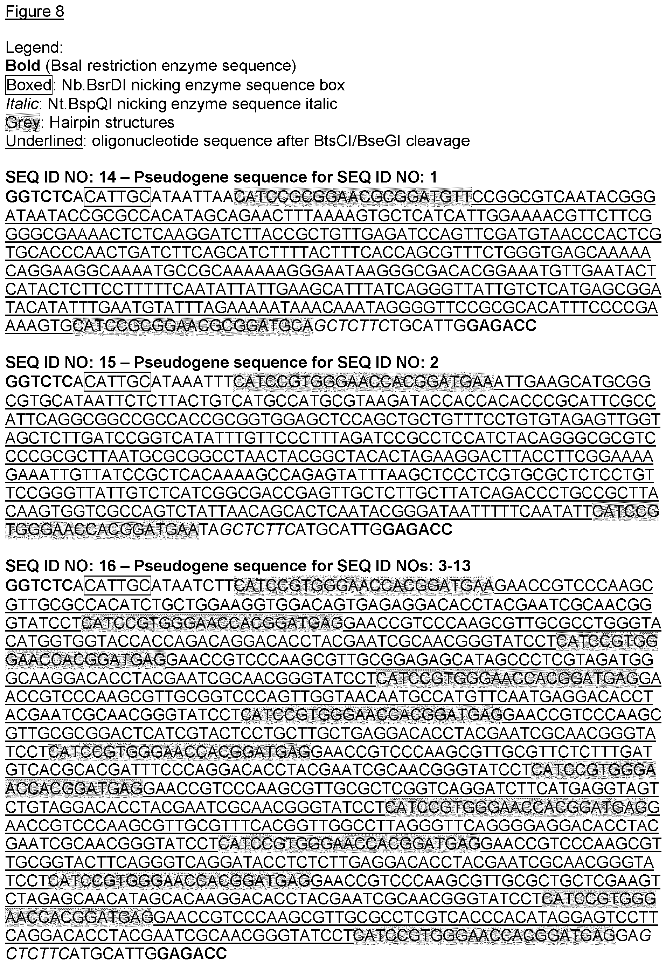

Figure 8 shows annotated versions of the pseudogene sequences that were used in the production of oligonucleotides having sequences corresponding to SEQ ID NOs: 1-13. The sequences recognised by the cleavage and nicking enzymes, the hairpin sequences, and final oligonucleotide sequences are identified.

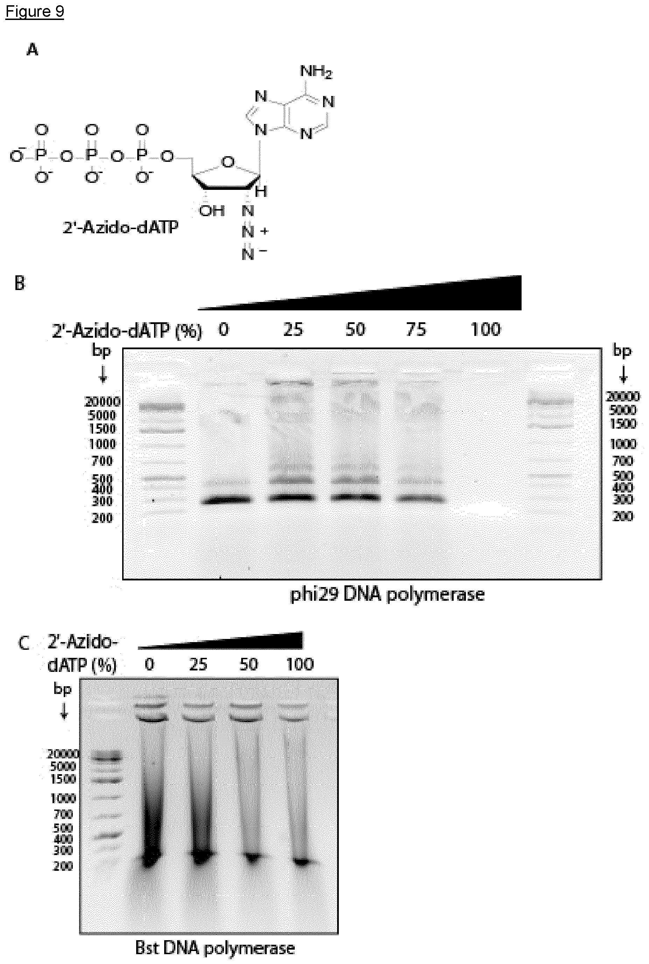

Figure 9 shows the structure of a 2'-Azido-dATP (A) and negative images of photographs of denaturing PAGE gels visualised using UV light following SybrGold staining (B and C). The PAGE gels show functionalised single stranded oligonucleotide products of the method of the invention comprising 2'-Azido-dATP produced using various relative amounts of 2'-Azido-dATP nucleotides, i.e. 25%, 50%, 75% and 100% and phi29 DNA polymerase (B) or Bst DNA polymerase (C).

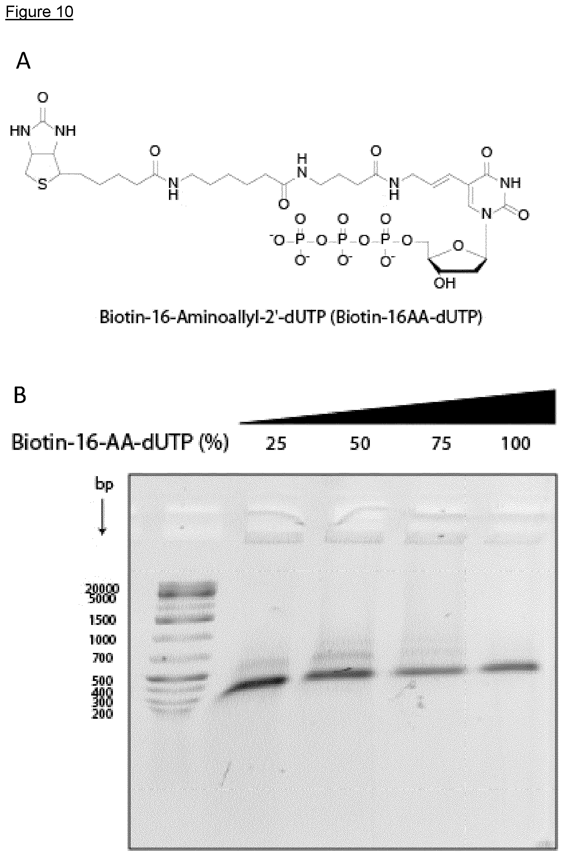

Figure 10 shows the structure of a Biotin-16-Aminoallyl-2'-dUTP (A) and a negative image of a photograph of a denaturing PAGE gel visualised using UV light following SybrGold staining (B). The PAGE gel shows functionalised single stranded oligonucleotide products of the method of the invention comprising Biotin-16-Aminoallyl-2'-dUTP produced using various relative amounts of Biotin-16-AA-dUTP nucleotides, i.e. 25%, 50%, 75% and 100% and phi29 DNA polymerase.

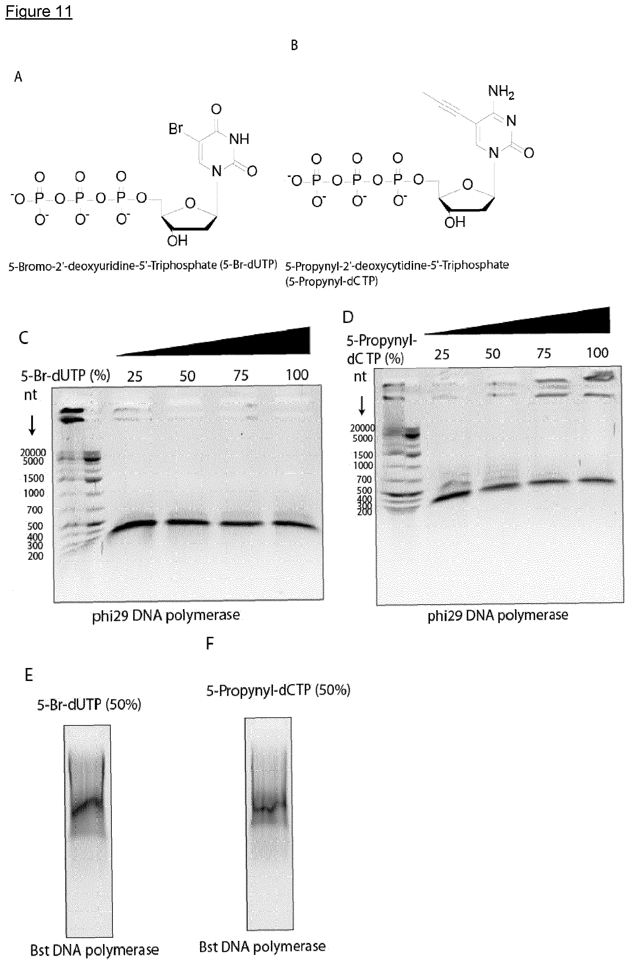

Figure 11 shows the structures of a 5'-Bromo-2'-deoxyuridine-5'Triphosphate nucleotide (A) and a 5'-Propynyl-2'-deoxycytidine-5'-Triphosphate nucleotide (B); negative images of photographs of denaturing PAGE gels visualised using UV light following SybrGold staining (C, D, E and F). The PAGE gels show functionalised single stranded oligonucleotide products of the method of the invention comprising 5'-Br-dUTP (C and E) or 5'-Propynyl-dCTP (D and F) produced using various relative amounts of the respective functionalised nucleotides, i.e. 25%, 50%, 75% and 100% and phi29 DNA polymerase or Bst DNA polymerase.

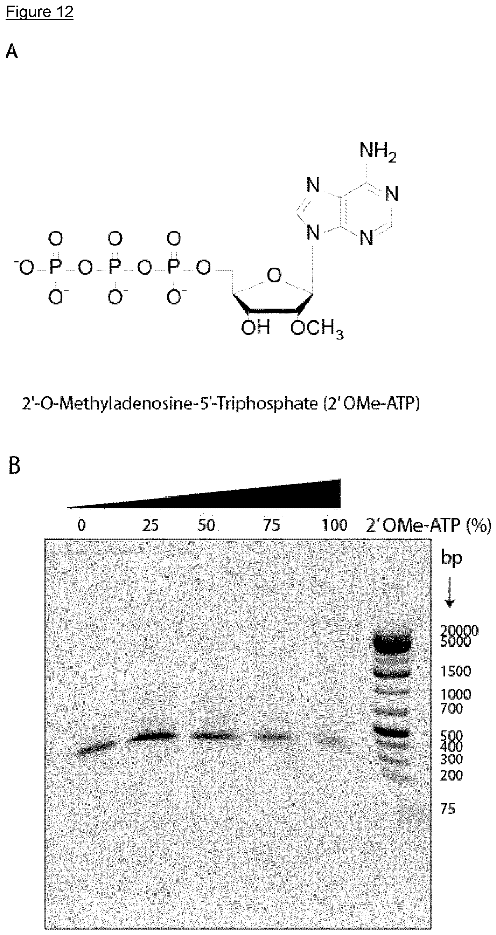

Figure 12 shows the structure of a 2'-O-Methyladenosine-5'-Triphosphate nucleotide (A) and a negative image of a photograph of a denaturing PAGE gel visualised using UV light following SybrGold staining (B). The PAGE gel shows functionalised single stranded oligonucleotide products of the method of the invention comprising 2'-OMe-ATP produced using various relative amounts of 2'-OMe-ATP nucleotides, i.e. 25%, 50%, 75% and 100% and phi29 DNA polymerase.

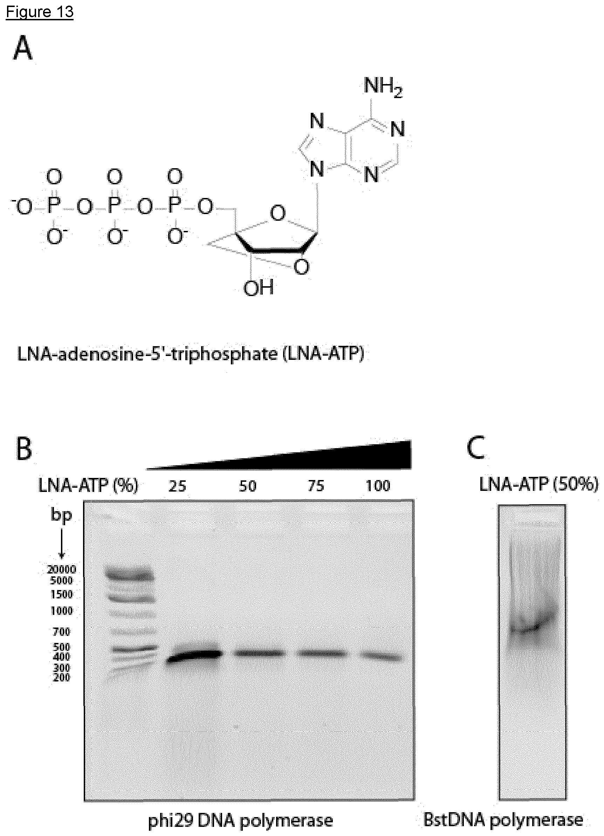

Figure 13 shows the structure of an LNA-adenosine-5'-triphosphate nucleotide (A) and negative images of photographs of denaturing PAGE gels visualised using UV light following SybrGold staining (B and C). The PAGE gels show functionalised single stranded oligonucleotide products of the method of the invention comprising LNA-ATP produced using various relative amounts of LNA-ATP nucleotides, i.e. 25%, 50%, 75% and 100% and phi29 DNA polymerase (B) or Bst DNA polymerase (C).

### Examples

### Example 1 - Enzymatic production of single stranded oligonucleotides comprising fluorescent nucleotides

Single stranded fluorescent oligonucleotides 378 nucleotides in length (SEQ ID NO: 1) were produced enzymatically using phi29 DNA polymerase. This was done via incorporation of two different functionalised dATP nucleobases, one comprising the fluorophore Cy3 (7-Propargylamino-7-deaza-ATP-Cy3) and one comprising the fluorophore ATTO-488 (7-Propargylamino-7-deaza-ATP-ATTO-488).

A double stranded circular DNA template containing SEQ ID NO: 1 and hairpin cleavage domains was prepared as described in Ducani et al., 2013, Nature Methods, 647-652. The template (1ng/µL) was nicked with Nb.BsrDI and Nt.BspQl (0.25 U/ µL) and a rolling circle amplification reaction (0.1-0.25ng/µL template DNA, phi29 DNA polymerase 0.25 U/µL, 0.1 µg T4 gene 32) was performed several times with different ratios of natural dATPs and functionalised dATPs in each reaction (i.e. different relative amounts of the functionalised dATP, 2%, 3% or 5%). The resulting RCA products were diluted five times in deionized water and 1X digestion buffer (50 mM Potassium Acetate, 20 mM Tris-acetate, 10 mM Magnesium Acetate, 100 µg/ml BSA, pH 7.9 at 25°C) and were then digested with BtsCI restriction enzyme (0.5U/µL) overnight at 50 °C and the digestion products were run on agarose gels. Imaging was done using the emission wavelengths corresponding to the two fluorophores, and after ethidium bromide staining, by UV visualization.

The resulting images are shown in Figures 1A and B for ATTO-488 and Cy3, respectively. It can be seen that increasing the percentage of dATP-ATTO-488 nucleotides resulted in oligonucleotides with higher fluorescence. However, the total amount of RCA product dropped by approximately 60% when 5% of the dATP nucleotides were dATP-ATTO-488.

Surprisingly and in contrast to the use of dATP-ATTO-488, the percentage of dATP-Cy3 nucleotides present did not seem to affect the efficiency of phi29 DNA polymerase; single stranded DNA products were visible with 5% of dATP-Cy3 in the RCA mixture (Figure 1B). Moreover, incorporation of the modified nucleotides did not prevent or reduce the efficiency of the BtsCI restriction enzyme, and consequently the release of the designed hairpins comprising the cleavage domains.

SEQ ID NO: 1:
CCGGCGTCAATACGGGATAATACCGCGCCACATAGCAGAACTTTAAAAGTGCTCATCAT
TGGAAAACGTTCTTCGGGGCGAAAACTCTCAAGGATCTTACCGCTGTTGAGATCCAGTT
CGATGTAACCCACTCGTGCACCCAACTGATCTTCAGCATCTTTTACTTTCACCAGCGTTT
CTGGGTGAGCAAAAACAGGAAGGCAAAATGCCGCAAAAAAGGGAATAAGGGCGACAC

GGAAATGTTGAATACTCATACTCTTCCTTTTTCAATATTATTGAAGCATTTATCAGGGTTA
TTGTCTCATGAGCGGATACATATTTGAATGTATTTAGAAAAATAAACAAATAGGGGTTCC
GCGCACATTTCCCCGAAAAGTG

### Example 2 - Enzymatic production of single stranded oligonucleotides comprising nucleotides with internalized alkyne groups, i.e. alkyne groups in the nucleobase

Single stranded oligonucleotides 420 nucleotides in length (SEQ ID NO: 2) comprising nucleotides with alkyne groups were produced enzymatically using phi29 DNA polymerase.

A double stranded circular DNA template containing SEQ ID NO: 2 and hairpin cleavage domains was prepared as described in Ducani et al., 2013, Nature Methods, 647-652. Nicking of the template RCA reactions were performed using the conditions described in Example 1, but using increasing relative amounts of 5'-Ethynyl-dUTP (5'-EdUTP), i.e. replacing dTTP with 5'-EdUTP such that the relative amount of 5'-EdUTP was 0% (control reaction), 25%, 50%, 75% or 100%. After amplification, the RCA products were digested by the BtsCI restriction enzyme and loaded on to an agarose gel as described in Example 2.

The results in Figure 2A surprisingly show that even when up 100% of the dTTP nucleotides were replaced with the alkyne functionalised dUTP, the RCA yields dropped only by 15-20%. Thus, Figure 2A also demonstrates that the RCA product was successfully and efficiently cleaved by BtsCl. The incorporation of the alkyne functionalised dUTP nucleotide was confirmed by the fact that a lower mobility of the functionalised oligonucleotide was observed.

An additional experiment was performed replacing phi29 DNA polymerase with Bst DNA polymerase. Gel electrophoresis showed no changes in amplification yield up to 50% of functionalised dUTP, with the final product shifted compared the one with 25% of modified dUTP, confirming the incorporation of the modified nucleotide which has higher molecular weight than its corresponding natural nucleotide (dTTP) (Figure 2B).

SEQ ID NO: 2:
ATTGAAGCATGCGGCGTGCATAATTCTCTTACTGTCATGCCATGCGTAAGATACCACCA
CACCCGCATTCGCCATTCAGGCGGCCGCCACCGCGGTGGAGCTCCAGCTGCTGTTTC
CTGTGTAGAGTTGGTAGCTCTTGATCCGGTCATATTTGTTCCCTTTAGATCCGCCTCCAT
CTACAGGGCGCGTCCCCGCGCTTAATGCGCGGCCTAACTACGGCTACACTAGAAGGA
CTTACCTTCGGAAAAGAAATTGTTATCCGCTCACAAAAGCCAGAGTATTTAAGCTCCCTC
GTGCGCTCTCCTGTTCCGGGTTATTGTCTCATCGGCGACCGAGTTGCTCTTGCTTATCA
GACCCTGCCGCTTACAAGTGGTCGCCAGTCTATTAACAGCACTCAATACGGGATAATTT
TTCAATATT

### Example 3 - Click chemistry reaction to conjugate azide-fluorophore to single stranded nucleotide comprising internalised alkyne groups

The successful incorporation of the functionalised 5-Ethynyl-dUTP nucleotides into the oligonucleotide produced in Example 2 was further demonstrated by performing a click chemistry reaction.

The functionalised oligonucleotide from the reaction with 75% of the alkyne functionalised dUTP nucleotide was incubated with a Cy3-azide (50 µM). The click chemistry solution also included copper sulfate (50 µM) as a catalyst, sodium ascorbate (50 mM) and THPTA (250 µM). As negative control, an oligonucleotide produced by the same method from the same template but with conventional dNTPs was also incubated with the Cy3-azide. In addition, the functionalised oligonucleotide comprising internalised alkyne groups was also incubated in the absence of the Cy3-azide. The reaction mixtures from the three reactions were run on an agarose gel and imaged (Figure 3). Fluorescent single stranded oligonucleotides of the expected length were observed only for the reaction comprising the functionalised oligonucleotide and the fluorophore-azide. In addition, no visible DNA degradation due to the presence of the copper sulfate was observed.

### Example 4 - Enzymatic production of single stranded oligonucleotides comprising endonuclease resistant nucleotides

2'-Fluoro-2'-deoxyuridine-5'-triphosphate (2'F-dUTP) or 2'-Deoxythymidine-5'-O-(1-Thiotriphosphate) (phosphorothioate dTTP) have both been previously used to modify DNA and RNA oligonucleotides for biomedicine and therapeutics applications, due to their capacity of conferring nuclease stability. These modified nucleotides were incorporated into single stranded DNA oligonucleotides by RCA using the experimental schemes described above. The conventional dTTP nucleotides were replaced by the functionalised nucleotides in increasing percentages from 0 to 100%. The tandem repeat RCA products were then digested by the BtsCI restriction enzyme into discrete 420 base single stranded functionalised oligonucleotides (SEQ ID NO: 2).

In the experiment performed with the 2'F-dUTP functionalised nucleotides, similar RCA yields were visible from 0% up to 75% of the functionalised nucleotide, with a drastic drop in yield observed with 100% of the functionalised nucleotide (Figure 4A).

In the phosphorothioate dTTP experiment, the RCA yield decreased gradually as the amount of the functionalised nucleotide was increased, up to approximately a 65% drop with 75% of the functionalised nucleotide, relative to the yield with only conventional nucleotides (Figure 4B).

However, overexposure of the agarose gels showed how functionalised single stranded oligonucleotides were produced even with 100% of functionalised nucleobases - see the panels on the right of Figures 4A and B.

Other phosphorothioate dNTPs (indicated as Alpha S dNTPs) were tested in additional experiments, either added one by one or in combination (Figure 4C). Even in the last case, in which 75% of all the conventional nucleotides were replaced with their corresponding Alpha S functionalised nucleotides, RCA products were synthesized and enzymatically cleaved to yield oligonucleotides visible on the agarose gel.

The endonuclease resistance of the functionalised oligonucleotides relative to control oligonucleotides produced with only conventional nucleotides was investigated. A control 420-nt long oligonucleotide produced with only conventional nucleotides, the 2'-F-dUTP functionalised oligonucleotides and phosphorothioate dTTP functionalised oligonucleotides (both produced using a relative amount of 75% of the functionalised nucleotide), were incubated with increasing concentrations of DNAse I (Figures 5A-C). The control oligonucleotide was completely digested with 18 mU/ml of DNAse I, but both the enzymatically produced 2'-F-dUTP and phosphorothioate dTTP functionalised DNA oligonucleotides were still visible on agarose gels after incubation with the same concentration of endonuclease.

### Example 5 - Enzymatic production of single stranded oligonucleotides comprising nucleotides with vinyl groups

Single stranded DNA oligonucleotides with lengths from 76-81 bases (SEQ ID NOs: 3 - 13), functionalised with the thymidine analogue 5-Vinyl-2'-deoxyuridine-5'-triphosphate (5-Vinyl-dUTP) were produced enzymatically via an RCA reaction according to the experimental schemes described above. All of the oligonucleotides were encoded on a single pseudogene (SEQ ID NO: 16). The incorporation of such a functionalised nucleotide in single stranded oligonucleotides enables copper-free click chemistry reactions to be used to conjugate tetrazine-like molecules to the oligonucleotide. This alkene-tetrazine reaction can be completely orthogonal to the alkyne-azide click chemistry reaction previously performed.

Increasing the amount of the functionalised nucleotide in the RCA reaction mixture, relative to the conventional nucleotide dTTP, led to the successful incorporation of the 5-Vinyl-dUTP into the single stranded RCA product and the successful digestion of the hairpin structures. However, the activity levels of both the phi29 DNA polymerase and the type II endonuclease used to cleave the RCA product were lower than in the absence of the functionalised nucleotide, which consequently led to higher molecular weight bands with undigested hairpin structures when the functionalised nucleotides fully replaced the conventional dTTP nucleotides (Figure 6).

| SEQ ID NO | Sequence |
|---|---|
| 3 | GAACCGTCCCAAGCGTTGCGCCACATCTGCTGGAAGGTGGAC AGTGAGAGGACACCTACGAATCGCAACGGGTATCCT |
| 4 | GAACCGTCCCAAGCGTTGCGCCTGGGTACATGGTGGTACCAC CAGACAGGACACCTACGAATCGCAACGGGTATCCT |
| 5 | GAACCGTCCCAAGCGTTGCGGAGAGCATAGCCCTCGTAGATG GGCAAGGACACCTACGAATCGCAACGGGTATCCT |
| 6 | GAACCGTCCCAAGCGTTGCGGTCCCAGTTGGTAACAATGCCA TGTTCAATGAGGACACCTACGAATCGCAACGGGTATCCT |
| 7 | GAACCGTCCCAAGCGTTGCGCGGACTCATCGTACTCCTGCTT GCTGAGGACACCTACGAATCGCAACGGGTATCCT |
| 8 | GAACCGTCCCAAGCGTTGCGTTCTCTTTGATGTCACGCACGAT TTCCCAGGACACCTACGAATCGCAACGGGTATCCT |
| 9 | GAACCGTCCCAAGCGTTGCGCTCGGTCAGGATCTTCATGAGG TAGTCTGTAGGACACCTACGAATCGCAACGGGTATCCT |
| 10 | GAACCGTCCCAAGCGTTGCGTTTCACGGTTGGCCTTAGGGTT CAGGGGAGGACACCTACGAATCGCAACGGGTATCCT |
| 11 | GAACCGTCCCAAGCGTTGCGGTACTTCAGGGTCAGGATACCT CTCTTGAGGACACCTACGAATCGCAACGGGTATCCT |
| 12 | GAACCGTCCCAAGCGTTGCGCTGCTCGAAGTCTAGAGCAACA TAGCACAAGGACACCTACGAATCGCAACGGGTATCCT |
| 13 | GAACCGTCCCAAGCGTTGCGCCTCGTCACCCACATAGGAGTC CTTCAGGACACCTACGAATCGCAACGGGTATCCT |

### Example 6 - Enzymatic production of single stranded oligonucleotides comprising thiolated nucleotides

Single stranded DNA oligonucleotides functionalised with a thiolated dTTP (4-Thiothymidine-5'-Triphosphate) were produced enzymatically via an RCA reaction using the reaction scheme and the templates described above (SEQ ID NOs: 3-13). All of the oligonucleotides were encoded on a single pseudogene (SEQ ID NO: 16)

The incorporation of the thiolated nucleotide into the single stranded oligonucleotide by phi29 DNA polymerase incorporation was very successful in amounts of the functionalised nucleotide up to 75% replacement of the corresponding conventional nucleotide (dTTP) (Figure 7), i.e. a relative amount of 75% of the functionalised nucleotide.

The RCA products were digested by type II restriction enzymes as described above, however, a complete digestion of the functionalised RCA products required an enzyme concentration 10 times higher than the concentration used on RCA products comprising only conventional nucleotides. When the conventional dTTP nucleotide was completely replaced with the functionalised thiolated nucleotide, no single stranded oligonucleotides were observed following treatment with the type II restriction enzymes, though there only very faint accumulation of undigested RCA products was observed in the well, suggesting that the activity of the polymerase was also affected.

### Example 7 - Enzymatic production of single stranded oligonucleotides comprising azide nucleotides

Single stranded oligonucleotides 420 nucleotides in length (SEQ ID NO: 2) comprising increasing percentages of functionalised 2'-Azido-dATP nucleotides (Figure 9A), which replace the corresponding conventional dATP nucleotides, were produced enzymatically using phi29 DNA polymerase (Figure 9B) and Bst DNA polymerase (Figure 9C).

The high density azido groups in the newly synthesized DNA strands enables the post-synthesis functionalization with alkyne molecules, either by a Cu(I)-catalyzed Huisgen cycloaddition ("click" chemistry), or by a strain-promoted [3 + 2] cycloaddition of azides and cycloalkynes, e.g. cyclooctyne or cyclononyne.

Two different polymerases with strand displacement activity were used in the amplification step: phi29 DNA polymerase or Bst DNA polymerase. Both polymerases were able to incorporate the functionalised nucleotide into the amplification products, which were then digested and run on an agarose gel.

DNA products produced with phi29 DNA polymerases were visible in the gel up to 75% of the modified nucleotides (Figure 9B) while Bst DNA polymerases products were visible up to 100% (Figure 9C), although Bst amplification buffer salts led to smear effect (even in the lane corresponding to 0% of 2'-Azido dATP). The functionalised nucleotides were incorporated successfully and did not significantly affect the formation of hairpin structures, which enable the cleavage of the amplification product.

### Example 8 - Enzymatic production of single stranded oligonucleotides comprising biotinylated nucleotides

Single stranded oligonucleotides 420 nucleotides in length (SEQ ID NO: 2) comprising increasing percentages (25%-100%) of functionalised Biotin-16-Aminoallyl-2'-dUTP (Figure 10A), which replace the corresponding conventional nucleotide dTTP, were produced enzymatically using phi29 DNA polymerase (Figure 10B).

The incorporation of the biotinylated nucleotides only slightly affected the DNA amplification reaction and, surprisingly, despite the potential for steric hindrance due to the large size of the functionalised nucleotide, did not interfere with the formation of the hairpin structures, which enable the cleavage of the RCA products. The incorporation of multiple internal biotins into a functionalised polynucleotide enables the conjugation with streptavidin functionalized molecules.

### Example 9 - Enzymatic production of single stranded oligonucleotides comprising 5-modified pyrimidines: 5-Bromo-2'-deoxyuridine-5'-Triphosphate and 5-Propynyl-2'-deoxycytidine-5'-Triphosphate

Single stranded oligonucleotides 420 nucleotides in length (SEQ ID NO: 2) comprising increasing percentages (25%-100%) of unnatural 5-modified pyrimidines: 5-Bromo-2'-deoxyuridine-5'-Triphosphate (Figure 11A) and 5-Propynyl-2'-deoxycytidine-5'-Triphosphate (Figure 11B), which replace the corresponding conventional nucleotides dTTP and dCTP, respectively, were produced enzymatically using phi29 DNA polymerase (Figures 11C and 11D) and Bst DNA polymerase (Figures 11E and 11F). Surprisingly, both functionalised nucleotides were successfully incorporated into the newly synthesized DNA sequence without affecting the formation of the hairpin structures in the RCA product, thus allowing the cleavage reaction to occur.

### Example 10 - Enzymatic production of single stranded oligonucleotides comprising 2'-O-Methyl-ATP

Single stranded oligonucleotides 420 nucleotides in length (SEQ ID NO: 2) comprising increasing percentages (25%-100%) of 2'-O-Methyl-ATP (Figure 12A), which replace the corresponding conventional nucleotide dATP, were produced enzymatically using phi29 DNA polymerase (Figure 12B). Even with 100% of the functionalised nucleotide present, the amplification product was still visible. This result was contrary to previous studies which showed that no known natural polymerases are capable of efficiently accepting these modified substrates (Romesberg, JACS 2004, 10.1021/ja038525p).

In addition, no higher molecular weight undigested DNA bands were visible in the gel, showing that the presence of the functionalised nucleotides did not affect the formation of the hairpin structure and its digestion by the restriction enzyme. This was a surprising result in view of the nuclease resistance which is conferred to DNA molecules by O-methyl groups.

### Example 11 Enzymatic production of single stranded oligonucleotides comprising LNA-adenosine-5'-triphosphate

Single stranded oligonucleotides 420 nucleotides in length (SEQ ID NO: 2) comprising increasing percentages (25%-100%) of LNA-adenosine-5'-triphosphate (Figure 13A), which replace the corresponding conventional nucleotide dATP, were produced enzymatically using phi29 DNA polymerase (Figure 13B) and Bst DNA polymerase (Figure 13C).

Although LNA monomers structurally mimic RNA, even with 100% of the functionalised nucleotide the efficiency of the polymerases, both phi29 DNA polymerase and Bst DNA polymerases, was not significantly affected. Moreover, the functionalised nucleotide did not interfere with the formation of the hairpin structures which enable digestion of the amplification products.

## Claims

1. A method for producing single stranded functionalised oligonucleotides, said method comprising:
(a) providing a circular DNA molecule comprising an oligonucleotide sequence bordered by cleavage domains that contain a sequence that is recognised by a cleavage enzyme, wherein a cleavage domain comprises a sequence capable of forming a hairpin structure, wherein the double-stranded portion of the hairpin structure comprises the sequence that is recognised by a cleavage enzyme;
(b) performing a rolling circle amplification (RCA) reaction with the circular DNA molecule of (a) as a template and one or more functionalised nucleotides (dNTPs);
(c) enzymatically cleaving the product of the RCA reaction at the cleavage domains to release the single stranded functionalised oligonucleotides; and
(d) isolating or purifying the single stranded functionalised oligonucleotides.

2. The method of claim 1, wherein the circular DNA molecule is double stranded and wherein the method comprises an additional step of cleaving a single strand of the circular DNA molecule to provide an RCA template, before the RCA reaction is performed, optionally wherein the step of cleaving a single strand of the circular DNA molecule to provide an RCA template comprises cleaving a single strand of the circular DNA molecule with a cleavage enzyme, preferably wherein the cleavage enzyme is a nickase, optionally wherein the cleavage enzyme is Nb.BsrDl, Nt.BspQl or a combination thereof, further preferably wherein the circular DNA molecule contains a sequence that is recognised by the cleavage enzyme, optionally wherein the sequence that is recognised by the cleavage enzyme is between the cleavage domains that border the oligonucleotide sequence and is not in the oligonucleotide sequence.

3. The method of claim 1 or 2, wherein the functionalised dNTPs are: (i) selected from the group consisting of: nucleotides comprising an alkyne group, fluorescently labelled nucleotides, nucleotides comprising a sterol group, nucleotides comprising a polyether group, nucleotides comprising a metal complex, nucleotides comprising a vinyl group, nucleotides comprising a thiol group, thionated nucleotides, nucleotides modified to have increased nuclease resistance, nucleotides comprising a chemical group capable of participating in a click chemistry reaction, and nucleotides that affect the thermostability of the oligonucleotide; and/or (ii) nucleotides comprising an alkyne group, an alkene group, an azide group, a halogen group, an O-methyl group, or a locked ribose sugar.

4. The method of any one of claims 1 to 3, wherein the functionalised dNTPs are nucleotides comprising an alkyne group, a vinyl group or an azide group.

5. The method of claim 3 or 4, wherein the method further comprises a step of conjugating a molecule or component to the oligonucleotide via the alkyne, vinyl or azide group, optionally wherein the molecule or component is selected from the group consisting of: a fluorophore, a sterol, a polyether, a metal complex, molecule containing a thiol group, a molecule containing a group providing increased nuclease resistance and a molecule containing a group capable of participating in a click chemistry reaction.

6. The method of any one of claims 1 to 5, wherein the cleavage domains: (i) are directly adjacent to the oligonucleotide sequence; and/or (ii) that border the oligonucleotide sequence are the same.

7. The method of any one of claims 1 to 6, wherein the cleavage enzyme is a type II restriction endonuclease, optionally a type IIS restriction endonuclease, such as BseGI or BtsCl.

8. The method of any one of claims 1 to 7, wherein the RCA reaction uses phi29 DNA polymerase or Bst DNA polymerase.

9. The method of any one of claims 1 to 8, wherein the circular DNA molecule comprises a plurality of oligonucleotide sequences, wherein each oligonucleotide sequence is bordered by cleavage domains, optionally wherein the oligonucleotide sequences are different.

10. The method of any one of claims 1 to 9, wherein step (a) comprises:
(i) cloning into a DNA plasmid a linear DNA molecule comprising the oligonucleotide sequence bordered by cleavage domains;
(ii) amplifying said plasmid, optionally comprising transfecting said DNA plasmid into bacteria and growing the bacteria;
(iii) excising part of the plasmid containing the DNA molecule comprising the oligonucleotide sequence bordered by cleavage domains; and
(iv) circularising the part of the plasmid obtained in step (iii),
optionally wherein the linear DNA molecule comprising the oligonucleotide sequence bordered by cleavage domains further comprises a 5' end region and a 3' end region each comprising a cleavage domain and wherein step (iii) comprises cleaving the cleavage domains in the end regions with a cleavage enzyme, optionally wherein said cleavage enzyme is BsmBl or Bsal.

11. The method of any one of claims 1 to 10, wherein the circular DNA molecule comprises a sequence between the cleavage domains that border the oligonucleotide sequence, which is not the oligonucleotide sequence, and which functions as: (a) an RCA primer binding site; and/or (b) a cleavage site for a nickase enzyme.

12. Use of a circular DNA molecule comprising an oligonucleotide sequence bordered by cleavage domains in the production of an isolated or purified single stranded functionalised oligonucleotide, wherein the cleavage domains contain a sequence that is recognised by a cleavage enzyme, and wherein a cleavage domain comprises a sequence capable of forming a hairpin structure, wherein the double-stranded portion of the hairpin structure comprises the sequence that is recognised by a cleavage enzyme.

13. The use of claim 12, wherein the cleavage domains are as defined in claim 6 or 7 and/or the DNA molecule is as defined in claim 9.

14. A kit for use in the method of any one of claims 1 to 11 comprising:
(i) a circular DNA molecule comprising an oligonucleotide sequence bordered by cleavage domains, wherein a cleavage domain comprises or consists of a sequence capable of forming a hairpin structure and wherein the double-stranded portion of the hairpin structure comprises a sequence that is recognised by a cleavage enzyme; and
(ii) functionalised dNTPs, optionally as defined in claim 3 or 4; and
(iii) one or more cleavage enzymes that cleave the cleavage domains of (i), optionally wherein the cleavage domains are as defined in claims 6 or 7 and/or the DNA molecule is as defined in claim 9.

15. The method of any one of claims 1 to 4 or 6 to 11, being a method for producing a pool of single stranded functionalised oligonucleotides for use in single molecule fluorescence in situ hybridisation (smFISH), wherein the functionalised nucleotides are fluorescently labelled nucleotides and wherein each single stranded functionalised oligonucleotide in the pool contains about 15-30, preferably about 20-25, nucleotides.

16. The method of any one of claims 1 to 11, being a method for producing a pool of single stranded functionalised oligonucleotides for use in single molecule fluorescence in situ hybridisation (smFISH), wherein the functionalised nucleotides are nucleotides comprising a chemical group capable of participating in click chemistry, and wherein the method further comprises a step of conjugating a fluorescent label to at least one functionalised nucleotide in each functionalised oligonucleotide via click chemistry, and wherein each single stranded functionalised oligonucleotide in the pool contains about 15-30, preferably about 20-25, nucleotides.

17. The method of claim 16, wherein: (i) the nucleotides comprising a chemical group capable of participating in click chemistry are nucleotides comprising an azide group, an alkyne group, an alkene group, a nitrone group, a tetrazine group or a tetrazole group, or a combination thereof; and/or (ii) the step of conjugating a fluorescent label to at least one functionalised nucleotide in each functionalised oligonucleotide via click chemistry is carried out before or after the functionalised oligonucleotides are hybridised to a nucleic acid molecule comprising a target sequence.

## Patentansprüche

1. Verfahren zum Herstellen einzelsträngiger funktionalisierter Oligonukleotide, wobei das Verfahren Folgendes umfasst:
(a) Bereitstellen eines zirkulären DNA-Moleküls, das eine Oligonukleotid-Sequenz umfasst, die von Spaltdomänen begrenzt ist, die eine Sequenz enthalten, die von einem Spaltenzym erkannt wird, wobei eine Spaltdomäne eine Sequenz umfasst, die im Stande ist, eine Haarnadelstruktur zu bilden, wobei der doppelsträngige Abschnitt der Haarnadelstruktur die Sequenz umfasst, die von einem Spaltenzym erkannt wird;
(b) Durchführen einer Rolling-Circle-Amplifikations- (RCA) Reaktion mit dem zirkulären DNA-Molekül von (a) als einer Vorlage und einem oder mehreren funktionalisierten Nukleotiden (dNTPs);
(c) enzymatisches Spalten des Produkts der RCA-Reaktion bei den Spaltdomänen, um die einzelsträngigen funktionalisierten Oligonukleotide freizusetzen; und
(d) Isolieren oder Reinigen der einzelsträngigen funktionalisierten Oligonukleotide.

2. Verfahren nach Anspruch 1, wobei das zirkuläre DNA-Molekül doppelsträngig ist und wobei das Verfahren einen zusätzlichen Schritt zum Spalten eines einzelnen Strangs des zirkulären DNA-Moleküls umfasst, um eine RCA-Vorlage bereitzustellen, bevor die RCA-Reaktion durchgeführt wird, wobei optional der Schritt zum Spalten eines einzelnen Strangs des zirkulären DNA-Moleküls, um eine RCA-Vorlage bereitzustellen, Spalten eines einzelnen Strangs des zirkulären DNA-Moleküls mit einem Spaltenzym umfasst, wobei bevorzugt das Spaltenzym eine Nickase ist, wobei optional das Spaltenzym Nb.BsrDI, Nt.BspQI oder eine Kombination davon ist, wobei bevorzugt weiter das zirkuläre DNA-Molekül eine Sequenz enthält, die das Spaltenzym erkennt, wobei optional die Sequenz, die das Spaltenzym erkennt, zwischen den Spaltdomänen liegt, die die Oligonukleotidsequenz begrenzen und nicht in der Oligonukleotidsequenz ist.

3. Verfahren nach Anspruch 1 oder 2, wobei die funktionalisierten dNTPs Folgendes sind: (i) aus der Gruppe ausgewählt, bestehend aus: Nukleotiden, die eine Alkyngruppe umfassen, fluoreszierend markierten Nukleotiden, Nukleotiden, die eine Sterolgruppe umfassen, Nukleotiden, die eine Polyethergruppe umfassen, Nukleotiden, die einen Metallkomplex umfassen, Nukleotiden, die eine Vinylgruppe umfassen, Nukleotiden, die eine Thiolgruppe umfassen, thionisierten Nukleotiden, Nukleotiden, die modifiziert sind, um erhöhte Nukleasenwiderstandsfähigkeit aufzuweisen, Nukleotiden, die eine chemische Gruppe umfassen, die dazu im Stande ist, an einer Click-Chemie-Reaktion teilzunehmen, und Nukleotiden, die die Wärmestabilität des Oligonukleotids beeinflussen; und/oder (ii) Nukleotiden, die eine Alkyngruppe, eine Alkengruppe, eine Azidgruppe, eine Halogengruppe, eine O-Methylgruppe oder einen blockierten Ribosezucker umfassen.

4. Verfahren nach einem der Ansprüche 1 bis 3, wobei die funktionalisierten dNTPs Nukleotide sind, die eine Alkyngruppe, eine Vinylgruppe oder eine Azidgruppe umfassen.

5. Verfahren nach Anspruch 3 oder 4, wobei das Verfahren weiter einen Schritt zum Konjugieren eines Moleküls oder einer Komponente mittels der Alkyn-, Vinyl- oder Azidgruppe an das Oligonukleotid umfasst, wobei optional das Molekül oder die Komponente aus der Gruppe ausgewählt ist, bestehend aus: einem Fluorphor, einem Sterol, einem Polyether, einem Metallkomplex, einem Molekül, das eine Thiolgrupe enthält, einem Molekül, das eine Gruppe enthält, die erhöhte Nukleasenwiderstandsfähigkeit bereitstellt, und einem Molekül, das eine Gruppe enthält, die dazu im Stande ist, an einer Click-Chemie-Reaktion teilzunehmen.

6. Verfahren nach einem der Ansprüche 1 bis 5, wobei die Spaltdomänen: (i) direkt an die Oligonukleotidsequenz angrenzen; und/oder (ii) die die Oligonukleotidsequenz begrenzen, dieselben sind.

7. Verfahren nach einem der Ansprüche 1 bis 6, wobei das Spaltenzym eine Typ-II-Restriktionsendonuklease, optional eine Typ-IIS-Restriktionsendonuklease, wie BseGI oder BtsCI ist.

8. Verfahren nach einem der Ansprüche 1 bis 7, wobei die RCA-Reaktion phi29 DNA-Polymerase oder Bst DNA-Polymerase verwendet.

9. Verfahren nach einem der Ansprüche 1 bis 8, wobei das zirkuläre DNA-Molekül eine Vielzahl von Oligonukleotidsequenzen umfasst, wobei jede Oligonukleotidsequenz von Spaltdomänen begrenzt ist, wobei optional die Oligonukleotidsequenzen unterschiedlich sind.

10. Verfahren nach einem der Ansprüche 1 bis 9, wobei Schritt (a) Folgendes umfasst:
(i) Klonen in ein DNA-Plasmid eines linearen DNA-Moleküls, das die Oligonukleotidsequenz umfasst, die von Spaltdomänen begrenzt ist;
(ii) Verstärken des Plasmids, optional umfassend Transfizieren des DNA-Plasmids in Bakterien und Aufwachsen der Bakterien;
(iii) Exzidieren eines Teils des Plasmids, der das DNA-Molekül enthält, das die Oligonukleotidsequenz umfasst, die von Spaltdomänen begrenzt ist; und
(iv) Zirkularisieren des Teils des Plasmids, der in Schritt (iii) erhalten wird, wobei optional das lineare DNA-Molekül, das die Oligonukleotidsequenz umfasst, die von Spaltdomänen begrenzt ist, weiter einen 5' Endbereich und einen 3' Endbereich umfasst, wobei jeder eine Spaltdomäne umfasst und wobei Schritt (iii) Spalten der Spaltdomänen in die Endbereiche mit einem Spaltenzym umfasst, wobei optional das Spaltenzym BsmBI oder Bsal ist.

11. Verfahren nach einem der Ansprüche 1 bis 10, wobei das zirkuläre DNA-Molekül eine Sequenz zwischen den Spaltdomänen umfasst, die die Oligonukleotidsequenz begrenzen, die nicht die Oligonukleotidsequenz ist, und die als Folgendes fungiert: (a) eine RCA-Primer-Bindungsstelle; und/oder (b) eine Spaltstelle für ein Nickaseenzym.

12. Verwendung eines zirkulären DNA-Moleküls, das eine Oligonukleotid-Sequenz umfasst, die von Spaltdomänen begrenzt ist, bei der Herstellung eines isolierten oder gereinigten einzelsträngigen funktionalisierten Oligonukleotids, wobei die Spaltdomänen eine Sequenz enthalten, die von einem Spaltenzym erkannt wird, und wobei eine Spaltdomäne eine Sequenz umfasst, die im Stande ist, eine Haarnadelstruktur zu bilden, wobei der doppelsträngige Abschnitt der Haarnadelstruktur die Sequenz umfasst, die von einem Spaltenzym erkannt wird.

13. Verwendung nach Anspruch 12, wobei die Spaltdomänen wie in Anspruch 6 oder 7 definiert sind und/oder das DNA-Molekül wie in Anspruch 9 definiert ist.

14. Kit zur Verwendung bei dem Verfahren nach einem der Ansprüche 1 bis 11, umfassend:
(i) ein zirkuläres DNA-Molekül, das eine Oligonukleotid-Sequenz umfasst, die von Spaltdomänen begrenzt ist, wobei eine Spaltdomäne eine Sequenz umfasst oder daraus besteht, die im Stande ist, eine Haarnadelstruktur zu bilden, und wobei der doppelsträngige Abschnitt der Haarnadelstruktur eine Sequenz umfasst, die von einem Spaltenzym erkannt wird; und
(ii) funktionalisierte dNTPs, optional wie in Anspruch 3 oder 4 definiert; und
(iii) ein oder mehrere Spaltenzyme, die die Spaltdomänen von (i) spalten, wobei optional die Spaltdomänen wie in den Ansprüchen 6 oder 7 definiert sind und/oder das DNA-Molekül wie in Anspruch 9 definiert ist.

15. Verfahren nach einem der Ansprüche 1 bis 4 oder 6 bis 11, das ein Verfahren zum Herstellen eines Pools einsträngiger funktionalisierter Oligonukleotide zur Verwendung in Einzelmolekülfluoreszenz in-situ-Hybridisierung (smFISH) ist, wobei die funktionalisierten Nukleotide fluoreszierend markierte Nukleotide sind und wobei jedes einzelsträngige funktionalisierte Oligonukleotid in dem Pool etwa 15-30, bevorzugt etwa 20-25 Nukleotide enthält.

16. Verfahren nach einem der Ansprüche 1 bis 11, das ein Verfahren zum Herstellen eines Pools einsträngiger funktionalisierter Oligonukleotide zur Verwendung in Einzelmolekülfluoreszenz-in-situ-Hybridisierung (smFISH) ist, wobei die funktionalisierten Nukleotide Nukleotide sind, die eine chemische Gruppe umfassen, die dazu im Stande ist, in Click-Chemie teilzunehmen, und wobei das Verfahren weiter einen Schritt zum Konjugieren einer fluoreszierenden Markierung an mindestens ein funktionalisiertes Nukleotid in jedem funktionalisierten Oligonukleotid mittels Click-Chemie umfasst und wobei jedes einzelsträngige funktionalisierte Oligonukleotid in dem Pool etwa 15-30, bevorzugt etwa 20-25 Nukleotide enthält.

17. Verfahren nach Anspruch 16, wobei: (i) die Nukleotide, die eine chemische Gruppe umfassen, die dazu im Stande ist, in Click-Chemie teilzunehmen, Nukleotide, die eine Azidgruppe, eine Alkyngruppe, eine Alkengruppe, eine Nitrongruppe, eine Tetrazingruppe oder eine Tetrazolgruppe umfassen, oder eine Kombination davon sind; und/oder (ii) der Schritt zum Konjugieren einer fluoreszierenden Markierung an mindestens ein funktionalisiertes Nukleotid in jedem funktionalisierten Oligonukleotid mittels Click-Chemie durchgeführt wird, bevor oder nachdem die funktionalisierten Oligonukleotide zu einem Nukleinsäuremolekül hybridisiert werden, das eine Zielsequenz umfasst.

## Revendications

1. Procédé de production d'oligonucléotides monocaténaires fonctionnalisés, ledit procédé comprenant :
(a) la fourniture d'une molécule d'ADN circulaire comprenant une séquence d'oligonucléotides flanquée de domaines de clivage qui contiennent une séquence qui est reconnue par une enzyme de clivage, dans lequel un domaine de clivage comprend une séquence apte à former une structure en épingle à cheveux, dans lequel la partie bicaténaire de la structure en épingle à cheveux comprend la séquence qui est reconnue par une enzyme de clivage ;
(b) la réalisation d'une réaction d'amplification en cercle roulant (RCA) avec la molécule d'ADN circulaire de (a) en tant que modèle et un ou plusieurs nucléotides fonctionnalisés (dNTP) ;
(c) le clivage enzymatique du produit de la réaction RCA aux domaines de clivage pour libérer les oligonucléotides monocaténaires fonctionnalisés ; et
(d) l'isolement ou la purification des oligonucléotides monocaténaires fonctionnalisés.

2. Procédé selon la revendication 1, dans lequel la molécule d'ADN circulaire est bicaténaire et dans lequel le procédé comprend une étape suppléme ntaire de clivage d'un simple brin de la molécule d'ADN circulaire pour fournir un modèle RCA, avant que la réaction RCA ne soit réalisée, éventuellement dans lequel l'étape de clivage d'un simple brin de la molécule d'ADN circulaire pour fournir un modèle RCA comprend le clivage d'un simple brin de la molécule d'ADN circulaire avec une enzyme de clivage, de préférence dans lequel l'enzyme de clivage est une nickase, éventuellement dans lequel l'enzyme de clivage est Nb.BsrDI, Nt.BspQl ou une combinaison de celles-ci, en outre de préférence dans lequel la molécule d'ADN circulaire contient une séquence qui est reconnue par l'enzyme de clivage, éventuellement dans lequel la séquence qui est reconnue par l'enzyme de clivage est comprise entre les domaines de clivage qui flanquent la séquence d'oligonucléotides et n'est pas dans la séquence d'oligonucléotides.

3. Procédé selon la revendication 1 ou 2, dans lequel les dNTP fonctionnalisés sont : (i) sélectionnés dans le groupe consistant en : des nucléotides comprenant un groupe alcyne, des nucléotides marqués par fluorescence, des nucléotides comprenant un groupe stérol, des nucléotides comprenant un groupe polyéther, des nucléotides comprenant un complexe métallique, des nucléotides comprenant un groupe vinyle, des nucléotides comprenant un groupe thiol, des nucléotides thionatés, des nucléotides modifiés pour présenter une résistance aux nucléases accrue, des nucléotides comprenant un groupe chimique apte à participer à une réaction de chimie click, et des nucléotides qui affectent la thermostabilité de l'oligonucléotide ; et/ou (ii) des nucléotides comprenant un groupe alcyne, un groupe alcène, un groupe azide, un groupe halogène, un groupe O-méthyle, ou un sucre ribose fermé.

4. Procédé selon l'une quelconque des revendications 1 à 3, dans lequel les dNTP fonctionnalisés sont des nucléotides comprenant un groupe alcyne, un groupe vinyle ou un groupe azide.

5. Procédé selon la revendication 3 ou 4, dans lequel le procédé comprend en outre une étape de conjugaison d'une molécule ou d'un composant à l'oligonucléotide via le groupe alcyne, vinyle ou azide, éventuellement dans lequel la molécule ou le composant est sélectionné dans le groupe consistant en : un fluorophore, un stérol, un polyéther, un complexe métallique, une molécule contenant un groupe thiol, une molécule contenant un groupe fournissant une résistance aux nucléases accrue et une molécule contenant un groupe apte à participer à une réaction de chimie click.

6. Procédé selon l'une quelconque des revendications 1 à 5, dans lequel les domaines de clivage : (i) sont directement adjacents à la séquence d'oligonucléotides ; et/ou (ii) qui flanquent la séquence d'oligonucléotides sont identiques.

7. Procédé selon l'une quelconque des revendications 1 à 6, dans lequel l'enzyme de clivage est une endonucléase de restriction de type II, éventuellement une endonucléase de restriction de type IIS, par exemple BseGI ou BtsCI.

8. Procédé selon l'une quelconque des revendications 1 à 7, dans lequel la réaction RCA utilise de l'ADN polymérase phi29 ou de l'ADN polymérase Bst.

9. Procédé selon l'une quelconque des revendications 1 à 8, dans lequel la molécule d'ADN circulaire comprend une pluralité de séquences d'oligonucléotides, dans lequel chaque séquence d'oligonucléotides est flanquée de domaines de clivage, éventuellement dans lequel les séquences d'oligonucléotides sont différentes.

10. Procédé selon l'une quelconque des revendications 1 à 9, dans lequel l'étape (a) comprend :
(i) le clonage en plasmide d'ADN d'une molécule d'ADN linéaire comprenant la séquence d'oligonucléotides flanquée de domaines de clivage ;
(ii) l'amplification dudit plasmide, éventuellement comprenant la transfection dudit plasmide d'ADN dans des bactéries et le développement des bactéries ;
(iii) l'ablation d'une partie du plasmide contenant la molécule d'ADN comprenant la séquence d'oligonucléotides flanquée des domaines de clivage ; et
(iv) la circularisation de la partie du plasmide obtenue à l'étape (iii), éventuellement dans lequel la molécule d'ADN linéaire comprenant la séquence d'oligonucléotides flanquée des domaines de clivage comprend en outre une région terminale 5' et une région terminale 3' comprenant chacune un domaine de clivage et dans lequel l'étape (iii) comprend le clivage des domaines de clivage dans les régions terminales avec une enzyme de clivage, éventuellement dans lequel ladite enzyme de clivage est BsmBI ou Bsal.

11. Procédé selon l'une quelconque des revendications 1 à 10, dans lequel la molécule d'ADN circulaire comprend une séquence comprise entre les domaines de clivage qui flanquent la séquence d'oligonucléotides, qui n'est pas la séquence d'oligonucléotides, et qui fonctionne en tant que : (a) site de liaison d'amorce RCA ; et/ou (b) site de clivage pour une enzyme nickase.

12. Utilisation d'une molécule d'ADN circulaire comprenant une séquence d'oligonucléotides flanquée de domaines de clivage dans la production d'un oligonucléotide monocaténaire fonctionnalisé isolé ou purifié, dans lequel les domaines de clivage contiennent une séquence qui est reconnue par une enzyme de clivage, dans laquelle un domaine de clivage comprend une séquence apte à former une structure en épingle à cheveux, et dans laquelle la partie bicaténaire de la structure en épingle à cheveux comprend la séquence qui est reconnue par une enzyme de clivage.

13. Utilisation selon la revendication 12, dans laquelle les domaines de clivage sont tels que définis en revendication 6 ou 7 et/ou la molécule d'ADN est telle que définie en revendication 9.

14. Kit pour utilisation dans le procédé selon l'une quelconque des revendications 1 à 11 comprenant :
(i) une molécule d'ADN circulaire comprenant une séquence d'oligonucléotides flanquée de domaines de clivage, dans lequel un domaine de clivage comprend ou consiste en une séquence apte à former une structure en épingle à cheveux et dans lequel la partie bicaténaire de la structure en épingle à cheveux comprend une séquence qui est reconnue par une enzyme de clivage ; et
(ii) des dNTP fonctionnalisés, éventuellement tels que définis en revendication 3 ou 4 ; et
(iii) une ou plusieurs enzymes de clivage qui clivent les domaines de clivage de (i), éventuellement dans lequel les domaines de clivage sont tels que définis en revendications 6 ou 7 et/ou la molécule d'ADN est telle que définie en revendication 9.

15. Procédé selon l'une quelconque des revendications 1 à 4 ou 6 à 11, étant un procédé de production d'un groupe d'oligonucléotides monocaténaires fonctionnalisés pour utilisation lors d'une hybridation in situ en fluorescence de molécule unique (smFISH), dans lequel les nucléotides fonctionnalisés sont des nucléotides marqués par fluorescence et dans lequel chaque oligonucléotide monocaténaire fonctionnalisé dans le groupe contient environ 15-30, de préférence environ 20-25, nucléotides.

16. Procédé selon l'une quelconque des revendications 1 à 11, étant un procédé de production d'un groupe d'oligonucléotides monocaténaires fonctionnalisés pour utilisation lors d'une hybridation in situ en fluorescence de molécule unique (smFISH), dans lequel les nucléotides fonctionnalisés sont des nucléotides comprenant un groupe chimique apte à participer à une chimie click, et dans lequel le procédé comprend en outre une étape de conjugaison d'un marqueur de fluorescence à au moins un nucléotide fonctionnalisé dans chaque oligonucléotide fonctionnalisé par l'intermédiaire d'une chimie click, et dans lequel chaque oligonucléotide monocaténaire fonctionnalisé dans le groupe contient environ 15-30, de préférence environ 20-25, nucléotides.

17. Procédé selon la revendication 16, dans lequel : (i) les nucléotides comprenant un groupe chimique apte à participer à une chimie click sont des nucléotides comprenant un groupe azide, un groupe alcyne, un groupe alcène, un groupe nitrone, un groupe tétrazine ou un groupe tétrazole, ou une combinaison de ceux-ci ; et/ou (ii) l'étape de conjugaison d'un marqueur de fluorescence à au moins un nucléotide fonctionnalisé dans chaque oligonucléotide fonctionnalisé via une chimie click est mise en oeuvre avant ou après hybridation des oligonucléotides fonctionnalisés à une molécule d'acide nucléique comprenant une séquence cible.
